# EUROPEAN PATENT SPECIFICATION

(11) **EP 1 038 008 B1**
(45) Date of publication and mention of the grant of the patent: **18.10.2006**
(21) Application number: 98962104.0
(22) Date of filing: 14.12.1998
(51) Int. Cl.: C12N 15/56, C12N 15/10, C12N 9/42, C12Q 1/68, A23K 1/165, D21H 17/00, D06M 16/00, C11D 3/386

(54) **METHODS FOR PRODUCING EGIII-LIKE ENZYMES**
VERFAHREN ZUR HERSTELLUNG EGIII-ÄHNLICHER ENZYME
PROCEDES DE PRODUCTION DES ENZYMES APPARENTEES A EGGIII

(30) Priority: 16.12.1997 US 991720
(43) Date of publication of application: 27.09.2000
(73) Proprietor: GENENCOR INTERNATIONAL, INC., Palo Alto, California 94304 (US)
(72) Inventor: BOWER, Benjamin, S., Pacifica, CA 94070 (US); FOWLER, Timothy, San Carlos, CA 94070 (US); PHILLIPS, Jay, Ian, Palo Alto, CA 94306 (US)
(74) Representative: Kiddle, Simon John
(86) International application number: PCT/US1998/026552
(87) International publication number: WO 1999/031255

(56) References cited:
- WO-A-94/21801
- WO-A-97/43409
- KITAMOTO N ET AL: "Molecular cloning, purification and characterization of two endo-1,4-beta-glucanases from Aspergillus oryzae KBN616." APPLIED MICROBIOLOGY AND BIOTECHNOLOGY, vol. 46, no. 5-6, 1996, pages 538-544, XP002104210
- WITTMANN S ET AL: "Purification and characterization of the CelB endoglucanase from Streptomyces lividans 66 and DNA sequence of the encoding gene." APPLIED AND ENVIRONMENTAL MICROBIOLOGY, vol. 60, no. 5, 1994, pages 1701-1703, XP002104211
- HENRISSAT B: "A CLASSIFICATION OF GLYCOSYL HYDROLASES BASED ON AMINO ACID SEQUENCE SIMILARITIES" BIOCHEMICAL JOURNAL, vol. 280, 1991, pages 309-316, XP002050142

## Description

### BACKGROUND OF THE INVENTION

### 1. Field of the Invention

The present invention is directed to methods of obtaining an EGIII like cellulases which share unique highly conserved regions with a known useful cellulase. More specifically, the present invention relates to a methods of obtaining EGIII cellulases from fungi and bacteria which are based on five important conserved amino acid sequences which are also present in EGIII.

### 2. State of the Art

Cellulases are enzymes which are capable of hydrolysis of the β-D-glucosidic linkages in celluloses. Cellulolytic enzymes have been traditionally divided into three major classes: endoglucanases, exoglucanases or cellobiohydrolases and β-glucosidases (Knowles, J. et al., (1987), *TIBTECH* 5, 255-261); and are known to be produced by a large number of bacteria, yeasts and fungi.

Primary among the applications that have been developed for the use of cellulolytic enzymes are those involving degrading (wood)cellulose pulp into sugars for (bio)ethanol production, textile treatments like 'stone washing' and 'biopolishing', and in detergent compositions. Thus, cellulases are known to be useful in the treatment of mechanical pulp (see e.g., PCT Publication No. WO 92/16687). Additionally, cellulases are known to be useful as a feed additive (see e.g., PCT Publication No. WO 91/04673) and in grain wet milling.

Of primary importance, however, cellulases are used in the treatment of textiles, i.e., in detergent compositions for assisting in the removal of dirt or grayish cast (see e.g., Great Britain Application Nos. 2,075,028, 2,095,275 and 2,094,826 which illustrate improved cleaning performance when detergents incorporate cellulase) or in the treatment of textiles prior to sale to improve the feel and appearance of the textile. Thus, Great Britain Application No. 1,358,599 illustrates the use of cellulase in detergents to reduce the harshness of cotton containing fabrics and cellulases are used in the treatment of textiles to recondition used fabrics by making their colors more vibrant (see e.g.. The Shizuoka Prefectural Hammamatsu Textile industrial Research Institute Report, Vol. 24, pp. 54-61 (1986)). For example, repeated washing of cotton containing fabrics results in a grayish cast to the fabric which is believed to be due to disrupted and disordered fibrils, sometimes called "pills", caused by mechanical action. This greyish cast is particularly noticeable on colored fabrics. As a consequence, the ability of cellulase to remove the disordered top layer of the fiber and thus improve the overall appearance of the fabric has been of value.

Thus, cellulases have been shown to be effective in many industrial processes. Accordingly, there has been a trend in the field to search for specific cellulase compositions or components which have particularly effective performance profiles with respect to one or more specific applications. In this light, cellulases produced (expressed) in fungi and bacteria have been subject of attention. For example, cellulase produced by certain fungi such as *Trichoderma spp.* (especially *Trichoderma longibrachiatum*) have been given much attention because a complete cellulase system capable of degrading crystalline forms of cellulose is readily produced in large quantities via fermentation procedures. This specific cellulase complex has been extensively analyzed to determine the nature of its specific components and the ability of those components to perform in industrial processes. For example, Wood et al., "Methods in Enzymology", 160, 25, pages 234 et seq. (1988), disclose that complete fungal cellulase systems comprise several different enzyme classifications including those identified as exo-cellobiohydrolases (EC 3.2.1.91) ("CBH"), endoglucanases (EC 3.2.1.4) ("EG"), and ß-glucosidases (EC 3.2.1.21) ("BG"). The fungal cellulase classifications of CBH, EG and BG can be further expanded to include multiple components within each classification. U.S. Patent No. 5,475,101 (Ward et al.) discloses the purification and molecular cloning of one particularly useful enzyme called EGIII which is derived from *Trichoderma longibrachiatum.*

PCT Publication No. WO 94/14953 discloses endoglucanases which are encoded by a nucleic acid which comprises any one of a series of DNA sequences, each having 20 nucleotides.

Ooi et al., *Curr. Genet.,* Vol. 18, pp. 217-222 (1990) disclose the cDNA sequence coding for endoglucanase F1-CMC produced by *Aspergillus aculeatus* which contains the amino acid strings NNLWG, ELMIW and GTEPFT. Sakamoto et al., *Curr. Genet.,* Vol. 27, pp. 435-439 (1995) discloses the cCNA sequence encoding the endoglucanase CMCase-1 From *Aspergillus kawachii* IFO 4308 which contains the amino acid strings ELMIW and GTEPFT. Ward et al., discloses the sequence of EGIII having the amino acid strings NNLWG, ELMIW and GTEPFT Additionally, two cellulase sequences, one from *Erwinia carotovara* and *Rhodothermus marinus* are disclosed in Saarilahti et al., *Gene,* Vol. 90, pp. 9-14 (1990) and Hreggvidsson et al., *Appl. Environ. Microb.,* Vol. 62, No. 8, pp. 3047-3049 (1996) which contain the amino acid string ELMIW. However, none of these references discloses or suggests that these amino acid strings have any particular relevance in identifying or isolating other cellulases, and particularly fail to suggest that such cellulases are obtainable from such diverse organisms as bacteria, Actinomycetes and other filamentous fungi.

Kitamoto et al (Appl. Microbiol Biotechnol., 46:538-544,1996) discloses the cloning of two endo-1,4-β-glucanases from A. oryzae using primers designed from other Aspergillus sequences.

Despite knowledge in the art related to many cellulase compositions having applications in some or all of the above areas, there is a continued need for new cellulase compositions having improved characteristics which are useful in, for example, treating textiles, as a component of detergent compositions, in the treatment of pulp and paper, food processing, and in the conversion of biomass. Thus, while there has been significant improvement in terms of the understanding of cellulase compositions and their activities, there remains a need for alternative cellulase compositions which retain the beneficial effects of known cellulase compositions. In response to this need, Applicants herein surprisingly discovered that novel microbial enzymes which are related to an enzyme known to be useful in industrial applications, i.e., EGIII, can be detected and obtained by virtue of the presence of unique conserved sequences therein.

### SUMMARY OF THE INVENTION

The present invention was the product of intensive research by the inventors hereof related to determining whether valuable enzymes could be detected by using a routine PCR techniques based on important and newly discovered conserved sequences found within the sequence of EGIII. Surprisingly, the inventors hereof discovered that these conserved sequences are found not only in EGIII, but also in enzymes derived from organisms from classifications as diverse as filamentous fungi, bacteria and Actinomycete. By utilizing the present invention, a number of genes encoding novel cellulases which are related to EGIII in that they possess the conserved regions described herein have been isolated.

Accordingly, in one aspect, the present invention provides a method for obtaining a gene encoding an EGIII like cellulase comprising the steps of:
(i) preparing genomic DNA from an organism of interest;
(ii) preparing a DNA primer encoding an amino acid string selected from the group consisting of one or more of the following:
   (a) Asn-Asn-(Leu/Phe/Lys/Ile)-Trp-Gly
   (b) Glu-(Leu/Phe/Ile)-Met-Ile-Trp
   (c) Gly-Thr-Glu-Pro-Phe-Thr;
   (d) (Ser/Tyr/Cys/Trp/Thr/Asn/Lys/Arg)-(Val/Pro)-(Lys/Ala)-(Ser/Ala)-(Tyr/Phe); and
   (e) Lys-Asn-Phe-Phe-Asn-Tyr;
(iii) amplifying the genomic DNA from step (i) by the polymerase chain reaction (PCR) using DNA primers encoding amino acid strings (a) and/or (d) and (c) and/or (e) under conditions suitable for producing one or more gene fragments from said genomic DNA;
(iv) selecting and pooling the gene fragments amplified in step (iii) and reamplifying using DNA primers encoding amino acid strings (a) and/or (d) plus (b), or using DNA primers encoding amino acid strings (c) and/or (e) plus (b) to produce one or more further gene fragments; and
(v) isolating from said genomic DNA all or part of the gene corresponding to one or more the further gene fragments produced in step (iv).

In one embodiment of this aspect of the Invention, the method above is utilized to detect an EGIII like enzyme and comprises labeling said DNA primer and mixing in said step (3) under conditions of standard stringency to permit hybridization of said DNA primer with a complementary sequence within the genomic DNA; and subsequent identification and isolation of the gene corresponding to said complementary sequence from said organism of interest which encodes an EGIII cellulase.

The cellulases identified by the methods the present invention may be employed for textile treatment, e.g., in laundry detergent or stonewashing compositions, in the reduction or biomass, in the production of feed additives or treatment of feed, in the treatment of wood pulp for the production of paper or pulp based products, and in the treatment of starch during grain wet milling or dry milling to facilitate the production of glucose, high fructose corn syrup and/or alcohol.

An advantage of the present invention is that a repeatable protocol has been discovered which permits rapid and simple isolation of genes encoding valuable cellulase enzymes.

### BRIEF DESCRIPTION OF THE DRAWINGS

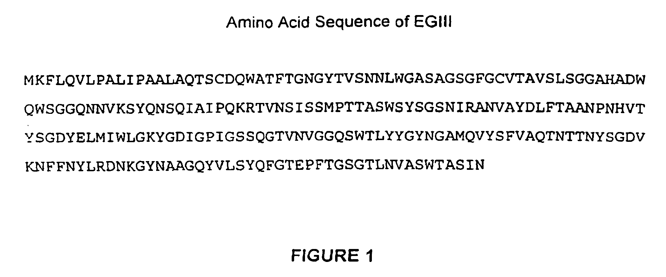

Fig. 1 illustrates the amino acid sequence of EGIII from *Trichoderma longibrachiatum.,*

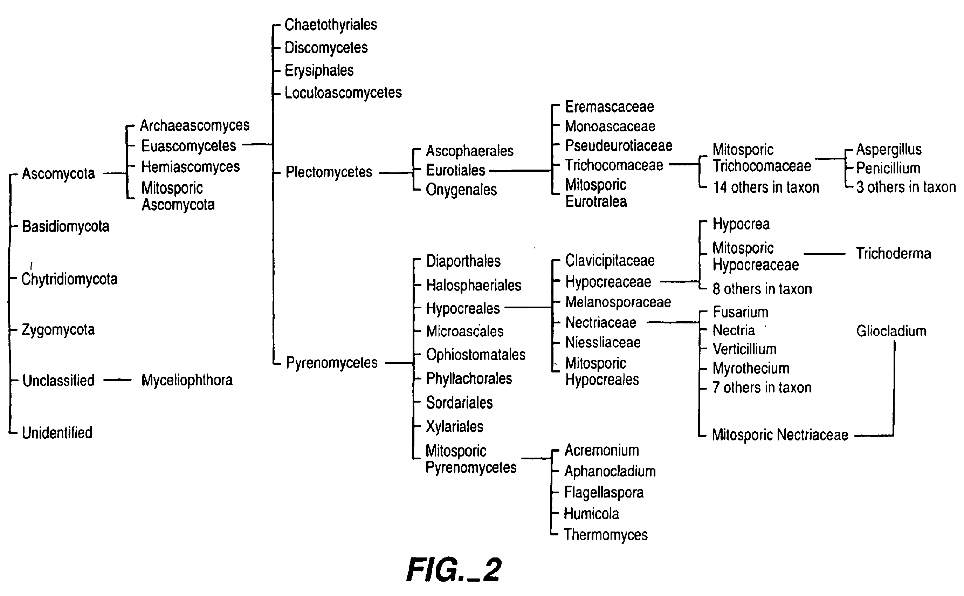

Fig. 2 illustrates branches of the fungal phylogenetic tree as interpreted by the NCBI.

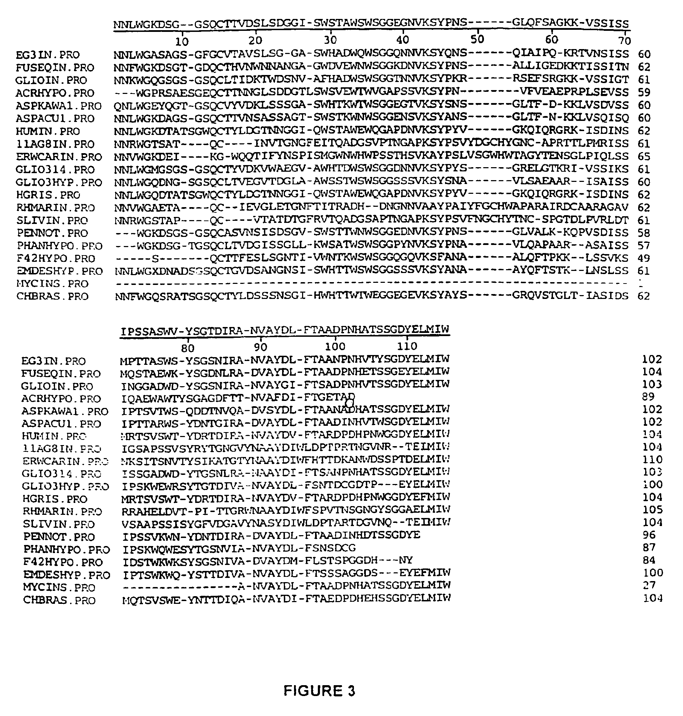

Fig. 3 illustrates a comparison of a 102 residue peptide taken from the sequence of EGIII with a corresponding peptide from *Fusarium equiseti [FUSEQIN]; Gliocladium roseum [GLIOIN]; Acremonium brachypenium [ACRHYPO]*(hypothetical protein sequence without intron); *Aspergillus kawachii [ASPKAWA1]; Aspergillus aculeatus [ASPACU1]; Humicola insolens [HUMIN]; Actinomycete (streptomyces) sp. 11AG8 [11AG8IN]; Erwinia carolovara [ERWCARIN]; Gliocladium roseum [GLIO314]; Gliocladium roseum [GLIOHYP]*(hypothetical protein sequence without intron); *Humicola grisea [HGRIS]; Rhodothermus marinus [RHMARIN]; Streptomyces lividans [SLIVINS]; Penicillium notaturn [PENNOT]; Phanerochaete chrysosporium [PHANHYPO]*(hypothetical protein sequence without intron); *Emericella desertoru [EMDESHYP]*(hypothetical protein sequence without intron); *Chaetomium brasillience* and *[CHBRAS]; Myceliopthora thermophila [MYCINS]*(only *27* amino acids).
Fig. 4 illustrates a chart showing the percent similarity of protein sequences compared in Fig. 3.

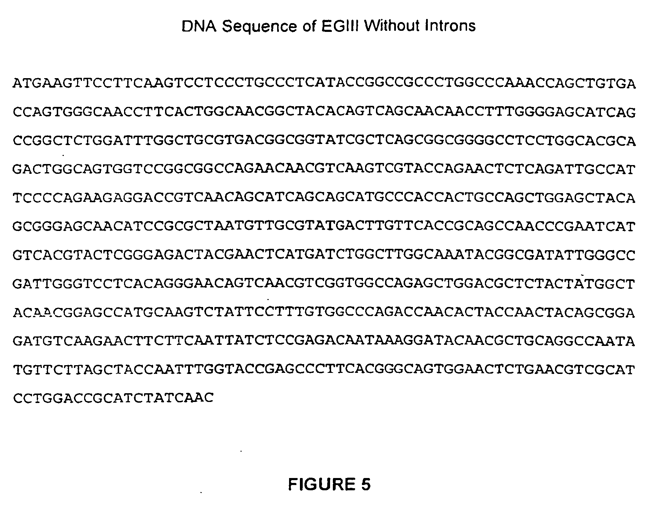

Fig. 5 illustrates the DNA sequence of EGIII from *Trichoderma longibrachiatum* without introns.

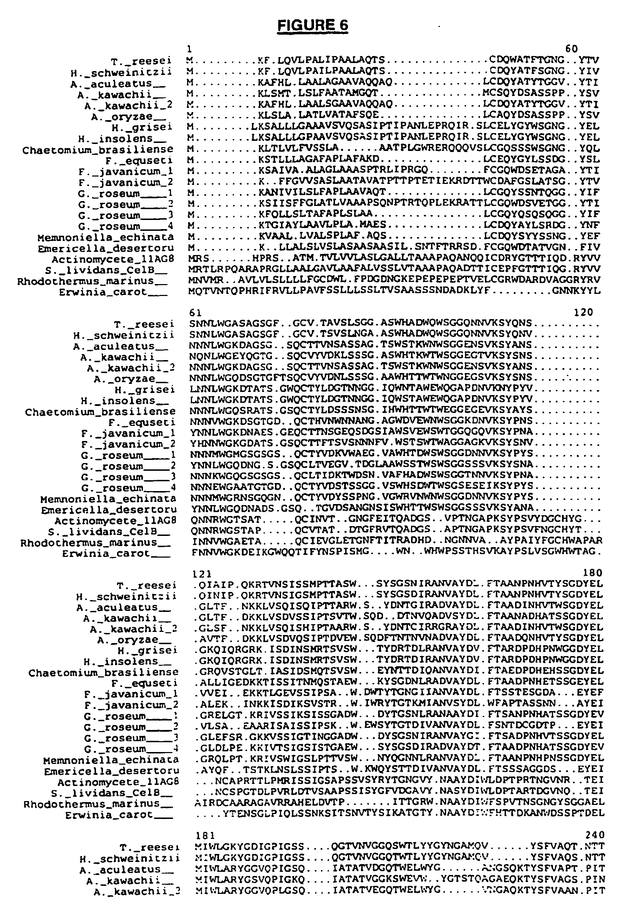

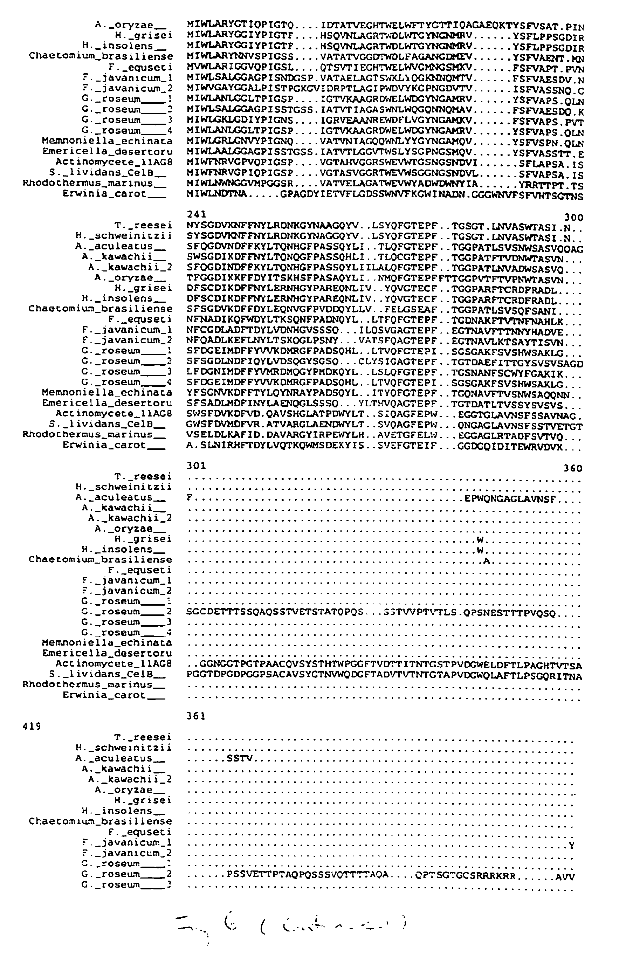

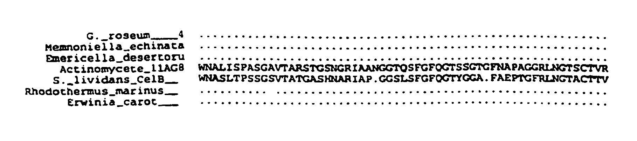

Fig. 6 illustrates an alignment of the full length sequence of 20 EGIII-like cellulases in alignment with EGIII, indicating equivalent residues based on primary sequence modeling, including those derived from *Trichoderma reesei, Hypocrea schweinitzii, Aspergillus aculeatus, Aspergillus kawachii (1), Aspergillus kawachii* (2), *Aspergillus oryzae, Humicola grisea, Humicola insolens, Chaetomium brasilliense, Fusarium equiseti, Fusarium javanicum (1), Fusarium javanicum* (2), *Gliocladium roseum (1), Gliocladium roseum* (2), *Gliocladium roseum* (3), *Gliocledium roseum (4), Memnoniella echinata, Emericella desertoru, Actinomycete 11AGB, Streptomyces lividans CelB, Rhodothermus marinus,* and *Erwinia caratovara.*

### DETAILED DESCRIPTION OF THE INVENTION

The present invention relates methods for obtaining genes which encode an EGIII like enzyme having cellulolytic activity and which is obtained from organisms, and more particularly from organisms other than *Trichoderma* spp., *Humicola spp.*, and *Erwinia carotovara* and *Rhodothermus marinus.*

Within the specification, certain terms are disclosed which are defined below so as to clarify the nature of the claimed invention.

"Cellulase" is a well classified category of enzymes in the art and includes enzymes capable of hydrolyzing cellulose polymers to shorter cellooligosaccharide oligomers, cellobiose and/or glucose. Common examples of cellulase enzymes include exo-cellobiohydrolases and endoglucanases and are obtainable from many species of cellulolytic organisms, particularly including fungi and bacteria.

"EGIII" cellulase refers to the endoglucanase component described in Ward et al., U.S. Patent No. 5,475,101 and Proceedings on the SecondTRICEL Symposium on *Trichoderma Reesei* Cellulases And Other Hydrolases, Suominen & Reinikainen eds., Espoo Finland (1993), pp. 153-158 (Foundation for Biotachnical and Industrial Fermentation Research, Vol. 8). As discussed therein, EGIII is derived from *Trichoderma reesei (longibrachiatum)* and is characterized by a pH optimum of about 5.8, an isoelectric point (pl) of from about 7.4 and a molecular weight of about 25 kD. The enzyme commonly referred to as EGII from *Trichoderma reesei* has been previously referred to in the literature by the nomenclature EGIII by some authors, but that enzyme differs substantially from the enzyme defined herein as EGIII in terms of molecular weight, pl and pH optimum.

"EG-III like enzyme", "EGIII like protein" or "EGIII like cellulase" according to the present invention means, on the one hand, an enzyme having cellulolytic activity which comprises an amino acid sequence comprising therein an amino acid string selected from the group consisting of one or more of:
(a) Asn-Asn-(Leu/Phe/Lys/Ile)-Trp-Gly
(b) Glu-(Leu/Phe/Ile)-Met-Ile-Trp
(c) Gly-Thr-Glu-Pro-Phe-Thr;
(d) (Ser/Tyr/Cys/Trp/Thr/Asn/Lys/Arg)-(Val/Pro)-(Lys/Ala)-(Ser/Ala)-(Tyr/Phe); and
(e) Lys-Asn-Phe-Phe-Asn-Tyr.
In one embodiment, the enzyme of the invention further has significant structural and/or sequence homology to EGIII. Thus, in one aspect of this embodiment of the invention, the enzyme has at least 30%, preferably at least 40% and most preferably at least 60% amino acid identity to EGIII. However, it should be recognized that homology alone is often not an appropriate measure for whether a particular enzyme identified by the methods described herein represents an EGIII like enzyme. Accordingly, while homologous enzymes are indeed detected by the methods described and exemplified herein, the degree of homology should not be seen as limiting the scope of the invention.

It is contemplated that the EGIII like enzymes of the invention may be found in many organisms which produce cellulases. However, likely sources of EGIII like enzyme include those derived from a bacterial or fungal sources, and more particularly, from an Actinomycete, a Bacillus or a filamentous fungus. In a preferred embodiment, the enzyme is derived from the filamentous fungal family Metazoa, preferably Euascomycetes. Within Metazoa, fungal phylogenetic classifications which produce EGIII like enzymes include the mitosporic Pyrenomycetes (including Acremonium), Sordariales (including Thielavia), Hypocreales (including Nectriaceae such as Fusarium, Necitia, Verticillium, Myrothecium and Gliocladium; and Hypocrea) and Eurotiales (including mitosporic Trichocomaceae such as Aspergillus and Penicillium).

The Euascomycete preferably belongs to Diaporthales, Halosphaeriales, Microascales, Ophiostomatales, Phyllachorales, Sordariales or Xylariales. Also preferably, the Eusacomycete belongs to Hypocreales comprising Clavicipitaceae, Melanosporaceae, Nectriaceae, Niessliaceae or Mitosporic Hypocreales. Further preferably, the Euascomycete belongs to Hypocreaceae, wherein said Hypocreaceae does not comprise Trichoderma. Most preferably, the Euascomycete is *Gliocladium spp., Fusarium spp., Acremonium spp., Myceliophtora spp., Verticillium spp., Myrothecium spp., Penicillium spp., Chaetomium spp., Emercella spp.,* and *Phanerochaete spp.* Specific organisms which are contemplated as possessing EGIII like enzymes include *Chaetomium thermophilum var therm., Chaetomium atrobrunneum, Chaetomium brasiliense, Chaetomium globosum, Chaetomium vitellium, Paecilomyces lilacinus, Chaetomium thermophilum var. dissitum, Humicola insolens, Humicola brevis, Memnonielia echinata, Fusarium equiseti, Fusarium oxysporum, fusarium stilboides, Myceliophthora thermophila, Fusarium javanicum, Humicola grisea var. thermoidea, Stibella thermophila, Melanocarpus albomyces, Arthrobotrys superba, Myceliophthora hinunilea, Chaetomium pachypodiodes, Myrothecium verrucaria, Penicillium crysogenum, Malbranchea sulfurea, Lunulospora curvula, Emericella desertorum, Acremonium strictum, Cylindrocarpon heteronema,* and *Ulocladium chartarum.*

Within the Actinomycetes, Streptomyces has been shown to possess EGIII like enzymes. Where the species of origin of the EGIII like cellulase is *Aspergillus,* the specific species is an *Aspergillus* comprising *A. aeneus, A. anthodesmis, A. aureofulgens, A. aureolatus, A. aveneceus, A. awamorii, A. bisporus, A. brunneouniseriatus, A. campestris, A. caesiellus, A. candidus, A. carbonarius, A. cameus, A. cervinus, A. clavatoflavus, A. clavatoanicus, A. clavatus, A. conicus, A. conjunctus, A. crustosus, A. deflectus, A. dimorphicus, A. ebumeocremeus, A. egyptiacus, A. ellipticus, A. elongatus, A. ficuum, A. flaschentraegeri, A. flavus, A. fumigatus, A. giganteus, A. glaucus, A. gorakhpurensis, A. gracilis, A. iizuke, A. itaconicus, A. japonicus, A. kambarensis, A. kanagawaensis, A. lanosus, A. leporis, A. longivesica, A. mellinus, A. multicolor, A. niger, A. nomius, A. nutans, A. ochraceus, A. pallidus, A. panamensis, A. parasiticus, A. parvulus, A. penicillioides, A. phialisepticus, A. phoenicis. A. proliferans, A.pulvinus, A.puniceus, A.raperi, A.recurvatus, A.restrictus, A.shirousami*, *A.sojae, A.sparsus, A. subolivaceus, A. subsessilis, A.tamarii, A.termus, A.terricola, A. thomii, A.tubingensis, A. unguis, A.unilateralis, A.ustus, A. versicolor, A.wentii, A.xerophilus*, *A.zonatus A.sp.*

As set out in the claims, EGIII like enzymes may be obtained according to methods that employ DNA primers which encode the following amino acid sequences:
(a) Asn-Asn-(Leu/Phe/Lys/Ile)-Trp-Gly
(b) Glu-(Leu/Phe/Ile)-Met-Ile-Trp
(c) Gly-Thr-Glu-Pro-Phe-Thr,
(d) (Ser/Tyr/Cys/Trp/Thr/Asn/Lys/Arg)-(Val/Pro)-(Lys/Ala)-(Ser/Ala)-(Tyr/Phe); and
(e) Lys-Asn-Phe-Phe-Asn-Tyr.

In the preferred embodiment according to this aspect of the invention, degenerate primers are prepared corresponding to the above peptides. The peptides are combined with a genomic DNA from a target organism (i.e., the organism in which the EGIII like enzyme is sought) under conditions suitable to initiate a standard PCR reaction. In this method, it is advantageous to select degenerate primers corresponding to peptides (a) and/or (d) plus primers corresponding to (c) and/or (e) and perform PCR with those peptides. After the PCR reaction has been performed, the resulting DNA is run on a polyacrylamide gel and bands corresponding in size to the EGIII fragment comprising peptides (a) and/or (d) in addition to (c) and/or (e), i.e., those in the 400-1000 base pair range, are selected out.
These fragments are pooled and reamplified using primers corresponding to peptides (a) and/or (d) plus primers corresponding to peptide (b) or, alternatively, using primers corresponding to peptide (c) and/or (e) plus primers corresponding to peptide (b). Strong bands of the expected size (in the case of EGIII like enzymes, the bands will correspond to the approximately 250-500 base pair range) are excised and sequenced. The sequence is then used to design exact match primers and these primers used with the technique referred to as rapid amplification of genomic DNA ends to obtain the full length gene, see e.g., Mizobuchi et al., *BioTechniques,* Vol. 15, No. 2, pp. 215-216 (1993).

In addition, it is also possible to use the degenerate DNA's as hybridization probes against a genomic library obtained from a target organism to analyze whether a given fragment correlates to a similar sequence in the target organism. A useful hybridization assay is as follows: Genomic DNA from a particular target source is fragmented by digestion with a restriction enzyme(s), e.g., EcoR I, Hind III, Barn HI. Cla I, Kpn I, Mlu I, Spe 1. Bgl II, Nco I, Xba I, Xho I and Xma I (supplied by New England Biolabs, Inc., Beverly, MA and Boehringer Mannheim) according to the manufacturer's instructions. The samples are then electrophoresed through an agarose gel (such as, for example, 0.7% agarose) so that separation of DNA fragments can be visualized by size. The gel may be briefly rinsed in distilled H₂O and subsequently depurinated in an appropriate solution (such as, for example. 0.25M HCl) with gentle shaking followed by denaturation for 30 minutes (in, for example, 0.4 M NaOH). A renaturation step may be included in which the gel is placed in 1.5 M NaCl, IM Tris, pH 7.0 with gentle shaking for 30 minutes. The DNA should then be transferred onto an appropriate positively charged membrane, for example the *Maximum Strength Nytran Plus* membrane (Schleicher & Schuell, Keene, N.H.), using a transfer solution (such as, for example, 6XSSC (900 mM NaCl, 90 mM trisodium citrate). After the transfer is complete, generally at about 2 hours or greater, the membrane is rinsed and air dried al room temperature after using a rinse solution (such as, for example, 2X SSC[2X SSC = 300 mM NaCl, 30 mM trisodium citrate]). The membrane should then be prehybridized, (for approximately 2 hours or more) in a suitable prehybridization solution (such as, for example, an aqueous solution containing per 100 mls: 30-50 mls formamide, 25 mls of 20X SSPE (1X SSPE = 0.18 M NaCl, 1 mM EDTA, 10 mM NaH₂PO₄, pH 7.7), 2.5 mls of 20% SDS, 1 ml of 10 mg/ml sheared herring sperm DNA).

A DNA probe corresponding to the peptide sequences above should be isolated by electrophoresis in an agarose gel, the fragment excised from the gel and recovered from the excised agarose. This purified fragment of DNA is then labeled (using, for example, the *Megaprime* labeling system according to the instructions of the manufacturer to incorporate P³² in the DNA (Amersham International plc, Buckinghamshire, England)). The labeled probe is denatured by heating to 95° C for 5 minutes and immediately added to the prehybridization solution above containing the membrane. The hybridization reaction should proceed for an appropriate time and under appropriate conditions, for example, for 18 hours at 37° C with gentle shaking. The membrane is rinsed (for example, in 2X SSC/0.3% SDS) and then washed with an appropriate wash solution and with gentle agitation. The stringency desired will be a reflection of the conditions under which the membrane (filter) is washed.

Specifically, the stringency of a given reaction (i.e., the degree of homology necessary for successful hybridization) will largely depend on the washing conditions to which the filter from the Southern Blot is subjected after hybridization. "Low-stringency" conditions as defined herein will comprise washing a filter from a Southern Blot with a solution of 0.2X SSC/0.1% SDS at 20° C for 15 minutes. Standard-stringency conditions comprise a further washing step comprising washing the filter from the Southern Blot a second time with a solution of 0.2X SSC/0.1% SDS at 37° C for 30 minutes.

The DNA which hybridizes with the DNA primers outlined above and thus identified by this method a corresponding EGIII encoding gene may be isolated by routine methods and used to express the corresponding EGIII like enzyme according to routine techniques. A preferred cloning procedure comprises the rapid amplification of genomic DNA ends described in, e.g., Mizobuchi et al., BioTechniques, Vol. 15, No. 2, pp. 215-216 (1993). Upon obtaining the cloned gene, routine methods for insertion of the DNA into a vector which can then be transformed into a suitable host cell are used. Culturing the transformed host cell under appropriate conditions then results in production of the EGIII like cellulase which can be obtained, purified and prepared as necessary for a particular application.

The EGIII like enzymes are preferably isolated or purified, In the context of the present invention, purification or isolation generally means that the EGIII like cellulase is altered from its natural state by virtue of separating the EGIII like cellulase from some or all of the naturally occurring substituents with which it is associated in nature, e.g., the source organism or other cellulases or enzymes expressed by the source organism in conjunction with the EGIII cellulase. Similarly, the EGIII like enzymes may be combined with other components which are not naturally present in the natural state. Isolation of purification may be accomplished by art recognized separation techniques such as ion exchange chromatography, affinity chromatography, hydrophobic separation, dialysis, protease treatment, ammonium sulphate precipitation or other protein salt precipitation techniques, centrifugation, size exclusion chromatography, filtration, microfiltration, gel electrophoresis or separation on a gradient to remove whole cells, cell debris, impurities, extraneous proteins, or enzymes undesired in the final composition.

"Cellulose containing fabric" means any sewn or unsewn fabrics, yarns or fibers made of cotton or non-cotton containing cellulose or cotton or non-cotton containing cellulose blends including natural cellulosics and manmade cellulosics (such as jute, flax, ramie, rayon, and lyocell). Included under the heading of manmade cellulose containing fabrics are regenerated fabrics that are well known in the art such as rayon. Other manmade cellulose containing fabrics include chemically modified cellulose fibers (e.g, cellulose derivatized by acetate) and solvent-spun cellulose fibers (e.g. lyocell). Specifically included within the definition of cellulose containing fabric is any yarn or fiber made of such materials. Cellulose containing materials are often incorporated into blends with materials such as synthetic fibers and natural non-cellulosic fibers such as wool and silk.

"Cotton-containing fabric" means sewn or unsewn fabrics, yarns or fibers made of pure cotton or cotton blends including cotton woven fabrics, cotton knits, cotton denims, cotton yarns, raw cotton and the like. When cotton blends are employed, the amount of cotton in the fabric is preferably at least about 35 percent by weight cotton. When employed as blends, the companion material employed in the fabric can include one or more non-cotton fibers including cellulosic or synthetic fibers such as polyamide fibers (for example, nylon 6 and nylon 66), acrylic fibers (for example, polyacrylonitrile fibers), and polyester fibers (for example, polyethylene terephthalate), polyvinyl alcohol fibers (for example, Vinylon), polyvinyl chloride fibers, polyvinylidene chloride fibers, polyurethane fibers, polyurea fibers and aramid fibers.

"Detergent composition" means a mixture which is intended for use in a wash medium for the laundering of soiled cellulose containing fabrics. In the context of the present invention, such compositions may include, in addition to cellulases and surfactants, additional hydrolytic enzymes, builders, bleaching agents, bleach activators, bluing agents and fluorescent dyes, caking inhibitors, masking agents, cellulase activators, antioxidants, and solubilizers. Such compositions are generally used for deaning soiled garments and are not used during the manufacturing process, in contrast to stonewashing compositions. Detergent compositions comprising cellulase are described in, for example. Clarkson et al., U.S. Patent No. 5,290,474 and EP Publication No. 271 004.

"Derivative" means a protein which is derived from a precursor protein (e.g., the native protein) by addition of one or more amino acids to either or both the C- and N-terminal end, substitution of one or more amino acids at one or a number of different sites in the amino acid sequence, deletion of one or more amino acids at either or both ends of the protein or at one or more sites in the amino acid sequence, or insertion of one or more amino acids at one or more sites in the amino acid sequence. The preparation of an enzyme derivative is preferably achieved by modifying a DNA sequence which encodes for the native protein, transformation of that DNA sequence into a suitable host, and expression of the modified DNA sequence to form the derivative enzyme. The derivative of the invention includes peptides comprising altered amino acid sequences in comparison with a precursor enzyme amino acid sequence (e.g., a wild type or native state enzyme), which peptides retain a characteristic enzyme nature of the precursor enzyme but which have altered properties in some specific aspect. For example, a cellulase derivative may have an increased pH optimum or increased temperature or oxidative stability but will retain its characteristic cellulolytic activity. Similarly, derivatives according to the present invention include a cellulose binding domain portions of which have either been added, removed or modified in such a way so as to significantly impair or enhance its cellulose binding ability. It is contemplated that derivatives according to the present invention may be derived from a DNA fragment encoding a cellulase derivative wherein the functional activity of the expressed cellulose derivative is retained. For example, a DNA fragment encoding a cellulase may further include a DNA sequence or portion thereof encoding a hinge or linker attached to the cellulase DNA sequence at either the 5' or 3' end wherein the functional activity of the encoded cellulase domain is retained. Derivative further includes chemical modification to change the characteristics of the enzyme.

"Expression vector" means a DNA construct comprising a DNA sequence which is operably linked to a suitable control sequence capable of effecting the expression of the DNA in a suitable host. Such control sequences may include a promoter to effect transcription, an optional operator sequence to control transcription, a sequence encoding suitable ribosome-binding sites on the mRNA, and sequences which control termination of transcription and translation. Different cell types are preferably used with different expression vectors. A preferred promoter for vectors used in *Bacillus subtilis* is the AprE promoter; a preferred promoter used in *E. coli* is the Lac promoter, a preferred promoter used in *Saccharomyces cerevisiae* is *PGK1,* a preferred promoter used in *Aspergillus niger* is *glaA,* and a preferred promoter for *Trichoderma reesei (longibrachiatum)* is *cbhl.* The vector may be a plasmid, a phage particle, or simply a potential genomic insert. Once transformed into a suitable host, the vector may replicate and function independently of the host genome, or may, under suitable conditions, integrate into the genome itself. In the present specification, plasmid and vector are sometimes used interchangeably. However, the invention is intended to include other forms of expression vectors which serve equivalent functions and which are, or become, known in the art. Thus, a wide variety of host/expression vector combinations may be employed in expressing the DNA sequences of this invention. Useful expression vectors, for example, may consist of segments of chromosomal, non-chromosomal and synthetic DNA sequences such as various known derivatives of SV40 and known bacterial plasmids, e.g., plasmids from *E. coli* including col E1, pCR1, pBR322, pMb9, pUC 19 and their derivatives, wider host range plasmids, e.g., RP4, phage DNAs e.g., the numerous derivatives of phage λ, e.g., NM989, and other DNA phages, e.g., M13 and filamentous single stranded DNA phages, yeast plasmids such as the 2µ plasmid or derivatives thereof, vectors useful in eukaryotic cells, such as vectors useful in animal cells and vectors derived from combinations of plasmids and phage DNAs, such as plasmids which have been modified to employ phage DNA or other expression control sequences. Expression techniques using the expression vectors of the present invention are known in the art and are described generally in, for example, Sambrook et al., *Molecular Cloning: A Laboratory Manual, Second Edition,* Cold Spring Harbor Press (1989). Often, such expression vectors including the DNA sequences of the invention are transformed into a unicellular host by direct insertion into the genome of a particular species through an integration event (see e.g., Bennett & Lasure, *More Gene Manipulations in Fungi,* Academic Press, San Diego, pp. 70-76 (1991) and articles cited therein describing targeted genomic insertion in fungal hosts).

"Host strain" or "host cell" means a suitable host for an expression vector comprising DNA according to the present invention. Host cells useful in the present invention are generally procaryotic or eucaryotic hosts, including any transformable microorganism in which expression can be achieved. Specifically, host strains may be *Bacillus subtilis, Escherichia coli, Trichoderma reesei (longibrachiatum), Saccharomyces cerevisiae* or *Aspergillus niger.* Host cells are transformed or transfected with vectors constructed using recombinant DNA techniques. Such transformed host cells are capable of both replicating vectors encoding swollenin and its variants (mutants) or expressing the desired peptide product. In a preferred embodiment according to the present invention, "host cell" means both the cells and protoplasts created from the cells of *Trichoderma sp.*

"Signal sequence" means a sequence of amino acids bound to the N-terminal portion of a protein which facilitates the secretion of the mature form of the protein outside of the cell. This definition of a signal sequence is a functional one. The mature form of the extracellular protein lacks the signal sequence which is cleaved off during the secretion process.

"DNA vector" means a nucleotide sequence which comprises one or more DNA fragments or DNA variant fragments encoding an EGIII like enzyme or derivatives described above which can be used, upon transformation into an appropriate host cell, to cause expression of the ECIII like cellulase.

"Functionally attached to" means that a regulatory region, such as a promoter, terminator, secretion signal or enhancer region is attached to a structural gene and controls the expression of that gene.

The present invention relates to the expression, purification and/or isolation and use of EGIII like enzymes and derivatives of such EGIII like enzyme. These enzymes are preferably prepared by recombinant methods utilizing the gene identified and isolated according to the methods described above. However, enzymes for use in the present invention may be obtained by other art recognized means such as purification from natural isolates.

It is conceived by the inventors that the microorganism to be transformed for the purpose of expressing an EGIII like enzyme may advantageously comprise a strain derived from *Trichoderma sp.* Thus, a preferred mode for preparing EGIII like enzymes according to the present invention comprises transforming a *Trichoderma sp.* host cell with a DNA construct comprising at least a fragment of DNA encoding a portion or all of the EGIII like enzyme detected as described above. The DNA construct will generally be functionally attached to a promoter. The transformed host cell is then grown under conditions so as to express the desired protein. Subsequently, the desired protein product is purified to substantial homogeneity.

However, it may in fact be that the best expression vehicle for a given DNA encoding an EGIII like enzyme may differ. Thus, it may be that it will be most advantageous to express a protein in a transformation host which bears phylogenetic similarity to the source organism for the EGIII like enzyme. Accordingly, the present description of a *Trichoderma spp.* expression system is provided for illustrative purposes only and as one option for expressing the EGIII like enzyme. One of skill in the art, however, may be inclined to express the DNA encoding EGIII like enzyme in a different host cell if appropriate and it should be understood that the source of the EGIII like enzyme should be considered in determining the optimal expression host. Additionally, the skilled worker in the field will be capable of selecting the best expression system for a particular gene through routine techniques utilizing the tools available in the art.

In one embodiment, the strain comprises *T. reesei (longibrachiatum)* which is a useful strain for obtaining overexpressed protein. For example. RL-P37, described by Sheir-Neiss et al. in *Appl. Microbiol. Biotechnology,* 20 (1984) pp. 46-53 is known to secrete elevated amounts of cellulase enzymes. Functional equivalents of RL-P37 include *Trichoderma reesei (longibrachiatum)* strain RUT-C30 (ATCC No. 56765) and strain QM9414 (ATCC No. 26921). It is contemplated that these strains would also be useful in overexpressing EGIII like enzymes.

Where it is desired to obtain the EGIII like cellulase in the absence of potentially detrimental native cellulolytic activity, it is useful to obtain a *Trichoderma* host cell strain which has had one or more cellulase genes deleted prior to introduction of a DNA construct or plasmid containing the DNA fragment encoding the EGIII like enzyme. Such strains may be prepared by the method disclosed in U.S. Patent No. 5,246,853 and WO 92/06209, which disclosures are hereby incorporated by reference. By expressing an EGIII like enzyme in a host microorganism that is missing one or more cellulase genes, the identification and subsequent purification procedures are simplified. Any gene from *Trichoderma sp.* which has been cloned can be deleted, for example, the *cbh1, cbh2, egl1,* and *egl3* genes as well as those encoding EGIII and/or EGV protein (see e.g., U.S. Patent No. 5,475,101 and WO 94/28117, respectively).

Gene deletion may be accomplished by inserting a form of the desired gene to be deleted or disrupted into a plasmid by methods known in the art. The deletion plasmid is then cut at an appropriate restriction enzyme site(s), internal to the desired gene coding region, and the gene coding sequence or part thereof replaced with a selectable marker. Flanking DNA sequences from the locus of the gene to be deleted or disrupted, preferably between about 0.5 to 2.0 kb, remain on either side of the selectable marker gene. An appropriate deletion plasmid will generally have unique restriction enzyme sites present therein to enable the fragment containing the deleted gene, including flanking DNA sequences, and the selectable marker gene to be removed as a single linear piece.

A selectable marker must be chosen so as to enable detection of the transformed fungus. Any selectable marker gene which is expressed in the selected microorganism will be suitable. For example, with *Trichoderma sp.,* the selectable marker is chosen so that the presence of the selectable marker in the transformants will not significantly affect the properties thereof. Such a selectable marker may be a gene which encodes an assayable product. For example, a functional copy of a *Trichoderma sp.* gene may be used which if lacking in the host strain results in the host strain displaying an auxotrophic phenotype.

In a preferred embodiment, a *pyr4*^{*-*} derivative strain of *Trichoderma sp.* is transformed with a functional *pyr4*^{*-*} gene, which thus provides a selectable marker for transformation. A *pyr4*^{*-*} derivative strain may be obtained by selection of *Trichoderma sp.* strains which are resistant to fluoroorotic acid (FOA). The *pyr4* gene encodes orotidine-5'-monophosphate decarboxylase, an enzyme required for the biosynthesis of uridine. Strains with an intact *pyr4* gene grow in a medium lacking uridine but are sensitive to fluoroorotic acid. It is possible to select *pyr4*^{*-*} derivative strains which lack a functional orotidine monophosphate decarboxylase enzyme and require uridine for growth by selecting for FOA resistance. Using the FOA selection technique it is also possible to obtain uridine requiring strains which lack a functional orotate pyrophosphoribosyl transferase. It is possible to transform these cells with a functional copy of the gene encoding this enzyme (Berges and Barreau, *Curr. Genet.,* 19, 1991, pp. 359-365). Selection of derivative strains is easily performed using the FOA resistance technique referred to above, and thus, the *pyr4* gene is preferably employed as a selectable marker.

To transform *pyr4*^{*-*} *Trichoderma sp.* so as to be lacking in the ability to express one or more cellulase genes, a single DNA fragment comprising a disrupted or deleted cellulase gene is then isolated from the deletion plasmid and used to transform an appropriate *pyr*^{*-*} *Trichoderma* host. Transformants are then identified and selected based on their ability to express the *pyr4* gene product and thus compliment the uridine auxotrophy of the host strain. Southern blot analysis is then carried out on the resultant transformants to identify and confirm a double crossover integration event which replaces part or all of the coding region of the genomic copy of the gene to be deleted with the *pyr4* selectable markers.

Although the specific plasmid vectors described above relate to preparation of *pyr*^{*-*} transformants, the present invention is not limited to these vectors. Various genes can be deleted and replaced in the *Trichoderma sp.* strain using the above techniques. In addition, any available selectable markers can be used, as discussed above. In fact, any *Trichoderma sp.* gene which has been cloned, and thus identified, can be deleted from the genome using the above-described strategy.

As stated above, the host strains used are derivatives of *Trichoderma sp.* which lack or have a nonfunctional gene or genes corresponding to the selectable marker chosen. For example, if the selectable marker of *pyr4* is chosen, then a specific *pyr4*^{*-*} derivative strain is used as a recipient in the transformation procedure. Similarly, selectable markers comprising *Trichoderma sp.* genes equivalent to the *Aspergillus nidulans* genes *amdS, argB, trpC, niaD* may be used. The corresponding recipient strain must therefore be a derivative strain such as *argB*^{*-*}*, trpC*^{*-*}*, niaD*^{*-*}*,* respectively.

DNA encoding the EGIII like enzyme is then prepared for insertion into an appropriate microorganism. According to the present invention, DNA encoding a EGIII like enzyme comprises all of the DNA necessary to encode for a protein which has functional cellulolytic activity. The DNA fragment or DNA variant fragment encoding the EGIII like enzyme or derivative may be functionally attached to a fungal promoter sequence, for example, the promoter of the *cbh1* or *egl1* gene.

It is also contemplated that more than one copy of DNA encoding a EGIII like enzyme may be recombined into the strain to facilitate overexpression. The DNA encoding the EGIII like cellulase may be prepared by the construction of an expression vector carrying the DNA encoding the cellulase. The expression vector carrying the inserted DNA fragment encoding the EGIII like cellulase may be any vector which is capable of replicating autonomously in a given host organism or of integrating into the DNA of the host, typically a plasmid. In preferred embodiments two types of expression vectors for obtaining expression of genes are contemplated. The first contains DNA sequences in which the promoter, gene coding region, and terminator sequence all originate from the gene to be expressed. Gene truncation may be obtained where desired by deleting away undesired DNA sequences (e.g., coding for unwanted domains) to leave the domain to be expressed under control of its own transcriptional and translational regulatory sequences. A selectable marker is also contained on the vector allowing the selection for integration into the host of multiple copies of the novel gene sequences.

The second type of expression vector is preassembled and contains sequences required for high level transcription and a selectable marker. It is contemplated that the coding region for a gene or part thereof can be inserted into this general purpose expression vector such that it is under the transcriptional control of the expression cassettes promoter and terminator sequences. For example, pTEX is such a general purpose expression vector. Genes or part thereof can be inserted downstream of the strong *cbh*1 promoter.

In the vector, the DNA sequence encoding the EGIII like enzyme of the present invention should be operably linked to transcriptional and translational sequences, i.e., a suitable promoter sequence and signal sequence in reading frame to the structural gene. The promoter may be any DNA sequence which shows transcriptional activity in the host cell and may be derived from genes encoding proteins either homologous or heterologous to the host cell. The signal peptide provides for extracellular production of the EGIII like enzyme or derivatives thereof. The DNA encoding the signal sequence is preferably that which is naturally associated with the gene to be expressed, however the signal sequence from any suitable source, for example an exo-cellobiohydrolase or endoglucanase from *Trichoderma,* is contemplated in the present invention.

The procedures used to ligate the DNA sequences coding for the EGIII like enzyme with the promoter, and insertion into suitable vectors are well known in the art.

The DNA vector or construct described above may be introduced in the host cell in accordance with known techniques such as transformation, transfection, microinjection, microporation, biolistic bombardment and the like.

In the preferred transformation technique, it must be taken into account that the permeability of the cell wall to DNA in *Trichoderma sp.* is very low. Accordingly, uptake of the desired DNA sequence, gene or gene fragment is at best minimal. There are a number of methods to increase the permeability of the *Trichoderma sp.* cell wall in the derivative strain (i.e., lacking a functional gene corresponding to the used selectable marker) prior to the transformation process.

The preferred method in the present invention to prepare *Trichoderma sp.* for transformation involves the preparation of protoplasts from fungal mycelium. The mycelium can be obtained from germinated vegetative spores. The mycelium is treated with an enzyme which digests the cell wall resulting in protoplasts. The protoplasts are then protected by the presence of an osmotic stabilizer in the suspending medium. These stabilizers include Sorbitol, mannitol, potassium chloride, magnesium sulfate and the like. Usually the concentration of these stabilizers varies between 0.8 M to 1.2 M. It is preferable to use about a 1.2 M solution of sorbitol in the suspension medium.

Uptake of the DNA into the host *Trichoderma sp.* strain is dependent upon the calcium ion concentration. Generally between about 10 mM CaCl₂ and 50 mM CaCl₂ is used in an uptake solution. Besides the need for the calcium ion in the uptake solution, other items generally included are a buffering system such as TE buffer (10 Mm Tris, pH 7.4; 1 mM EDTA) or 10 mM MOPS, pH 6.0 buffer (morpholinepropanesulfonic acid) and polyethylene glycol (PEG). It is believed that the polyethylene glycol acts to fuse the cell membranes thus permitting the contents of the medium to be delivered into the cytoplasm of the *Trichoderma sp.* strain and the plasmid DNA is transferred to the nucleus. This fusion frequently leaves multiple copies of the plasmid DNA tenderly integrated into the host chromosome.

Usually a suspension containing the *Trichoderma sp.* protoplasts or cells that have been subjected to a permeability treatment at a density of 10⁸ to 10⁹/ml, preferably 2 x 10⁸/ml are used in transformation. A volume of 100 microliters of these protoplasts or cells in an appropriate solution (e.g., 1.2 M sorbitol; 50 mM CaCl₂) are mixed with the desired DNA. Generally a high concentration of PEG is added to the uptake solution. From 0.1 to 1 volume of 25% PEG 4000 can be added to the protoplast suspension. However, it is preferable to add about 0.25 volumes to the protoplast suspension. Additives such as dimethyl sulfoxide, heparin, spermidine, potassium chloride and the like may also be added to the uptake solution and aid in transformation.

Generally, the mixture is then incubated at approximately 0°C for a period of between 10 to 30 minutes. Additional PEG is then added to the mixture to further enhance the uptake of the desired gene or DNA sequence. The 25% PEG 4000 is generally added in volumes of 5 to 15 times the volume of the transformation mixture; however, greater and lesser volumes may be suitable. The 25% PEG 4000 is preferably about 10 times the volume of the transformation mixture. After the PEG is added, the transformation mixture is then incubated at room temperature before the addition of a sorbitol and CaCl₂ solution. The protoplast suspension is then further added to molten aliquots of a growth medium. This growth medium permits the growth of transformants only. Any growth medium can be used in the present invention that is suitable to grow the desired transformants. However, if *Pyr*^{*+*} transformants are being selected it is preferable to use a growth medium that contains no uridine. The subsequent colonies are transferred and purified on a growth medium depleted of uridine.

At this stage, stable transformants may be distinguished from unstable transformants by their faster growth rate and the formation of circular colonies with a smooth, rather than ragged outline on solid culture medium lacking uridine. Additionally, in some cases a further test of stability may made by growing the transformants on solid non-selective medium (i.e. containing uridine), harvesting spores from this culture medium and determining the percentage of these spores which will subsequently germinate and grow on selective medium lacking uridine.

In a particular embodiment of the above method, the EGIII like enzymes or derivatives thereof are recovered in active form from the host cell after growth in liquid media either as a result of the appropriate post translational processing of the novel EGIII like enzyme or derivatives thereof.

The expressed EGIII like enzyme may be recovered from the medium by conventional techniques including separations of the cells from the medium by centrifugation, filtration, and precipitation of the proteins in the supernatant or filtrate with a salt, for example, ammonium sulphate. Additionally, chromatography procedures such as ion exchange chromatography or affinity chromatography may be used. Antibodies (polyclonal or monoclonal) may be raised against the natural purified EGIII like enzyme, or synthetic peptides may be prepared from portions of the EGIII like enzyme molecule and used to raise polyclonal antibodies.

Treatment of textiles according to the present invention contemplates textile processing or cleaning with a composition comprising a cellulase. Such treating includes, but is not limited to, stonewashing, modifying the texture, feel and/or appearance of cellulose containing fabrics or other techniques used during manufacturing or cleaning/reconditioning of cellulose containing fabrics. Additionally, treating within the context of this invention contemplates the removal of "immature" or "dead" cotton, from cellulosic fabric or fibers. Immature cotton is significantly more amorphous than mature cotton and results in a lesser quality fabric when present due to, for example, uneven dyeing. The composition contemplated in the present invention further includes a cellulase component for use in washing of a soiled manufactured cellulose containing fabric. For example, the cellulase may be used in a detergent composition for washing laundry. Detergent compositions useful in accordance with the present invention include special formulations such as pre-wash, pre-soak and home-use color restoration compositions. Such treating compositions, as described herein, may be in the form of a concentrate which requires dilution or in the form of a dilute solution or form which can be applied directly to the cellulose containing fabric. General treatment techniques for cellulase treatment of textiles are described in, for example, EP Publication No. 220 016 and GB Application Nos. 1,368,599 and 2,095,275.

Treatment of a cellulosic material according to the present invention further contemplates the treatment of animal feed, pulp and/or paper, food and grain for purposes known in the art. For example, cellulase is known to increase the value of animal feed, improve the drainability of wood pulp, enhance food products and reduce fiber in grain during the grain wet milling process or dry milling process.

Treating according to the instant invention comprises preparing an aqueous solution which contains an effective amount of cellulase together with other optional ingredients including, for example, a buffer, a surfactant, and/or a scouring agent. An effective amount of cellulase enzyme composition is a concentration of cellulase enzyme sufficient for its intended purpose. Thus, for example, an "effective amount" of cellulase in a stonewashing composition according to the present invention is that amount which will provide the desired effect, e.g., to produce a worn and faded look in the seams and on fabric panels. Similarly, an "effective amount" of cellulase in a composition intended for improving the feel and/or appearance of a cellulose containing fabric is that amount which will produce measurable improvements in the feel, e.g., improving the smoothness of the fabric, or appearance, e.g., removing pills and fibrils which tend to reduce the sharpness in appearance of a fabric. The amount of cellulase employed is also dependent on the equipment employed, the process parameters employed (the temperature of the cellulase treatment solution, the exposure time to the cellulase solution, and the like), and the cellulase activity (e.g., a particular solution will require a lower concentration of cellulase where a more active cellulase composition is used as compared to a less active cellulase composition). The exact concentration of cellulase in the aqueous treatment solution to which the fabric to be treated is added can be readily determined by the skilled artisan based on the above factors as well as the desired result. In stonewashing processes, it has generally been preferred that the cellulase be present in the aqueous treating solution in a concentration of from about 0.5 to 5,000 ppm and most preferably about 10 to 200 ppm total protein. In compositions for the improvement of feel and/or appearance of a cellulose containing fabric, it has generally been preferred that the cellulase be present in the aqueous treating solution in a concentration of from about 0.1 to 2000 ppm and most preferably about 0.5 to 200 ppm total protein.

In a preferred treating embodiment, a buffer is employed in the treating composition such that the concentration of buffer is sufficient to maintain the pH of the solution within the range wherein the employed cellulase exhibits activity which, in turn, depends on the nature of the cellulase employed. The exact concentration of buffer employed will depend on several factors which the skilled artisan can readily take into account. For example, in a preferred embodiment, the buffer as well as the buffer concentration are selected so as to maintain the pH of the final cellulase solution within the pH range required for optimal cellulase activity. The determination of the optimal pH range of the cellulases of the invention can be ascertained according to well known techniques. Suitable buffers at pH within the activity range of the cellulase are well known to those skilled in the art in the field.

In addition to cellulase and a buffer, the treating composition may optionally contain a surfactant. Suitable surfactants include any surfactant compatible with the cellulase and the fabric including, for example, anionic, non-ionic and ampholytic surfactants. Suitable anionic surfactants for use herein include linear or branched alkylbenzenesulfonates; alkyl or alkenyl ether sulfates having linear or branched alkyl groups or alkenyl groups; alkyl or alkenyl sulfates; olefinsulfonates; alkanesulfonates and the like. Suitable counter ions for anionic surfactants include alkali metal ions such as sodium and potassium; alkaline earth metal ions such as calcium and magnesium; ammonium ion; and alkanolamines having 1 to 3 alkanol groups of carbon number 2 or 3. Ampholytic surfactants include quaternary ammonium salt sulfonates, and betaine-type ampholytic surfactants. Such ampholytic surfactants have both the positive and negative charged groups in the same molecule. Nonionic surfactants generally comprise polyoxyalkylene ethers, as well as higher fatty acid alkanolamides or alkylene oxide adduct thereof, and fatty acid glycerine monoesters. Mixtures of surfactants can also be employed in manners known to those skilled in the art.

A concentrated cellulose composition can be prepared for use in the methods described herein. Such concentrates contain concentrated amounts of the cellulase composition described above, buffer and surfactant, preferably in an aqueous solution. When so formulated, the cellulase concentrate can readily be diluted with water so as to quickly and accurately prepare cellulase preparations having the requisite concentration of each constituent. When aqueous concentrates are formulated, these concentrates can be diluted so as to arrive at the requisite concentration of the components in the cellulase solution as indicated above. As is readily apparent, such cellulase concentrates will permit facile formulation of the cellulase solutions as well as permit feasible transportation of the composition to the location where it will be used. The treating concentrate can be in any art recognized form, for example, liquid, emulsion, gel, or paste. Such forms are well known to those skilled in the art.

When a solid cellulase concentrate is employed, the cellulose composition may be a granule, a powder, an agglomerate or a solid disk. The granules can be formulated so as to contain materials to reduce the rate of dissolution of the granules into the wash medium. Such materials and granules are disclosed in U.S. Patent No. 5,254,283.

Other materials can also be used with or placed in the cellulase composition of the present invention as desired, including stones, pumice, fillers, solvents, enzyme activators, and anti-redeposition agents depending on the eventual use of the composition.

By way of example, stonewashing methods will be described in detail, however, the parameters described are readily modified by the skilled artisan for other applications. i.e., improving the feel and/or appearance of a fabric. The cellulose containing fabric is contacted with the cellulase containing stonewashing composition containing an effective amount of the reilulase by intermingling the treating composition with the stonewashing composition, and thus bringing the cellulase enzyme into proximity with the fabric Subsequently, the aqueous solution containing the cellulase and the fabric is agitated. If the treating composition is an aqueous solution, the fabric may be directly soaked in the solution. Similarly, where the stonewashing composition is a concentrate, the concentrate is diluted into a water bath with the cellulose containing fabric. When the stonewashing composition is in a solid form, for example a pre-wash gel or solid stick, the stonewashing composition may be contacted by directly applying the composition to the fabric or to the wash liquor.

The cellulose containing fabric is incubated with the stonewashing solution under conditions effective to allow the enzymatic action to confer a stonewashed appearance to the cellulose containing fabric. For example, during stonewashing, the pH, liquor ratio, temperature and reaction time may be adjusted to optimize the conditions under which the stonewashing composition acts. "Effective conditions" necessarily refers to the pH, liquor ratio, and temperature which allow the cellulase enzyme to react efficiently with cellulose containing fabric, in this case to produce the stonewashed effect. However, such conditions are readily ascertainable by one of skill in the art. The reaction conditions effective for the stonewashing compositions of the present invention are substantially similar to well known methods used with corresponding prior art cellulase compositions. Accordingly, it is within the skill of those in the art to maximize conditions for using the stonewashing compositions.

The liquor ratios during stonewashing, i.e., the ratio of weight of stonewashing composition solution (i.e., the wash liquor) to the weight of fabric, employed herein is generally an amount sufficient to achieve the desired stonewashing effect in the denim fabric and is dependent upon the process used. Preferably, the liquor ratios are from about 4:1 to about 50:1; more preferably from about 5:1 to about 20:1, and most preferably from about 10:1 to about 15:1.

Reaction temperatures during stonewashing with the present stonewashing compositions are governed by two competing factors. Firstly, higher temperatures generally correspond to enhanced reaction kinetics, i.e., faster reactions, which permit reduced reaction times as compared to reaction times required at lower temperatures. Accordingly, reaction temperatures are generally at least about 10°C and greater. Secondly, cellulase is a protein which loses activity beyond a given reaction temperature, which temperature is dependent on the nature of the cellulase used. Thus, if the reaction temperature is permitted to go too high, the cellulolytic activity is lost as a result of the denaturing of the cellulase. While standard temperatures for cellulase usage in the art are generally in the range of 35° C to 65° C, which conditions would also be expected to be suitable for the cellulase of the invention, the optimal temperature conditions should be ascertained according to well known techniques with respect to the specific cellulase used.

Reaction times are dependent on the specific conditions under which the stonewashing occurs. For example, pH, temperature and concentration of cellulase will all effect the optimal reaction time. Generally, reaction times are from about 5 minutes to about 5 hours, and preferably from about 10 minutes to about 3 hours and, more preferably, from about 20 minutes to about 1 hour.

According to yet another preferred embodiment of the present invention, the cellulase of the invention may be employed in a detergent composition. The detergent compositions according to the present invention are useful as pre-wash compositions, pre-soak compositions, or for cleaning during the regular wash or rinse cycle. Preferably, the detergent composition of the present invention comprises an effective amount of cellulase, a surfactant, and optionally includes other ingredients described below.

An effective amount of cellulase employed in the detergent compositions of this invention is an amount sufficient to impart the desirable effects known to be produced by cellulase on cellulose containing fabrics, for example, depilling, softening, anti-pilling, surface fiber removal, anti-graying and cleaning. Preferably, the cellulase in the detergent composition is employed in a concentration of from about 10 ppm to about 20,000 ppm of detergent.

The concentration of cellulase enzyme employed in the detergent composition is preferably selected so that upon dilution into a wash medium, the concentration of cellulase enzyme is in a range of about 0.01 to about 1000 ppm, preferably from about 0.02 ppm to about 500 ppm, and most preferably from about 0.5 ppm to about 250 ppm total protein. The amount of cellulase enzyme employed in the detergent composition will depend on the extent to which the detergent will be diluted upon addition to water so as to form a wash solution.

The detergent compositions of the present invention may be in any art recognized form, for example, as a liquid, in granules, in emulsions, in gels, or in pastes. Such forms are well known to the skilled artisan. When a solid detergent composition is employed, the cellulase is preferably formulated as granules. Preferably, the granules can be formulated so as to additionally contain a cellulase protecting agent. The granule can be formulated so as to contain materials to reduce the rate of dissolution of the granule into the wash medium. Such materials and granules are disclosed in U.S. Patent No. 5,254,283.

The detergent compositions of this invention employ a surface active agent, i.e., surfactant, including anionic, non-ionic and ampholytic surfactants well known for their use in detergent compositions.

Suitable anionic surfactants for use in the detergent composition of this invention include linear or branched alkylbenzenesulfonates; alkyl or alkenyl ether sulfates having linear or branched alkyl groups or alkenyl groups; alkyl or alkenyl sulfates; olefinsulfonates; and alkanesul-fonates. Suitable counter ions for anionic surfactants include alkali metal ions such as sodium and potassium; alkaline earth metal ions such as calcium and magnesium: ammonium ion; and alkanolamines having 1 to 3 alkanol groups of carbon number 2 or 3. Ampholytic surfactants include quaternary ammonium salt sulfonates, and betaine-type ampholytic surfactants. Such ampholytic surfactants have both the positive and negative charged groups in the same molecule. Nonionic surfactants generally comprise polyoxyalkylene ethers, as well as higher fatty acid alkanolamides or alkylene oxide adduct thereof, fatty acid glycerine monoesters, and the like. Suitable surfactants for use in this invention are disclosed in British Patent Application No. 2 094 826 A. Mixtures of such surfactants can also be used. The surfactant or a mixture of surfactants is generally employed in the detergent compositions of this invention in an amount from about 1 weight percent to about 95 weight percent of the total detergent composition and preferably from about 5 weight percent to about 45 weight percent of the total detergent composition. In addition to the cellulase composition and the surfactant(s), the detergent compositions of this invention can optionally contain one or more of the following components:

### Hydrolases Except Cellulase

Suitable hydrolases include carboxylate ester hydrolase, thioester hydrolase, phosphate monoester hydrolase, and phosphate diester hydrolase which act on the ester bond; glycoside hydrolase which acts on glycosyl compounds; an enzyme that hydrolyzes N-glycosyl compounds; thioether hydrolase which acts on the ether bond; and a-amino-acyl-peptide hydrolase, peptidyl-amino acid hydrolase, acyl-amino acid hydrolase, dipeptide hydrolase, and peptidyl-peptide hydrolase which act on the peptide bond. Preferable among them are carboxylate ester hydrolase, glycoside hydrolase, and peptidyl-peptide hydrolase. Suitable hydrolases include (1) proteases belonging to peptidyl-peptide hydrolase such as pepsin, pepsin B, rennin, trypsin, chymotrypsin A, chymotrypsin B, elastase, enterokinase, cathepsin C, papain, chymopapain, ficin, thrombin, fibrinolysin, renin, subtilisin, aspergillopeptidase A, collagenase, clostridiopeptidase B, kallikrein, gastrisin, cathepsin D., bromelin, keratinase, chymotrypsin C, pepsin C, aspergillopeptidase B, urokinase, carboxypeptidase A and B, and aminopeptidase; (2) glycoside hydrolases (cellulase which is an essential ingredient is excluded from this group) α-amylase, β-amylase, gluco amylase, invertase, lysozyme, pectinase, chitinase, and dextranase. Preferably among them are α-amylase and β-amylase. They function in acid to neutral systems, but one which is obtained from bacteria exhibits high activity in an alkaline system; (3) carboxylate ester hydrolase including carboxyl esterase, lipase, pectin esterase, and chlorophyllase. Especially effective among them is lipase.

The hydrolase other than cellulase is incorporated into the detergent composition as much as required according to the purpose. It should preferably be incorporated in an amount of 0.001 to 5 weight percent, and more preferably 0.02 to 3 weight percent, in terms of purified protein. This enzyme should be used in the form of granules made of crude enzyme alone or in combination with other components in the detergent composition. Granules of crude enzyme are used in such an amount that the purified enzyme is 0.001 to 50 weight percent in the granules. The granules are used in an amount of 0.002 to 20 and preferably 0.1 to 10 weight percent. As with cellulases, these granules can be formulated so as to contain an enzyme protecting agent and a dissolution retardant material.

### Cationic Surfactants and Long-Chain Fatty Acid Salts

Such cationic surfactants and long-chain fatty acid salts include saturated or unsaturated fatty acid salts, alkyl or alkenyl ether carboxylic acid salts, a-sulfofatty acid salts or esters, amino acid-type surfactants, phosphate ester surfactants, quaternary ammonium salts including those having 3 to 4 alkyl substituents and up to 1 phenyl substituted alkyl substituents. Suitable cationic surfactants and long-chain fatty acid salts are disclosed in British Patent Application No. 2 094 826 A, the disclosure of which is incorporated herein by reference. The composition may contain from about 1 to about 20 weight percent of such cationic surfactants and long-chain fatty acid salts.

### Builders

### A. Divalent sequestering agents.

The composition may contain from about 0 to about 50 weight percent of one or more builder components selected from the group consisting of alkali metal salts and alkanolamine salts of the following compounds: phosphates, phosphonales, phosphonocarboxylates, salts of amino acids, aminopolyacetates high molecular electrolytes, non-dissociating polymers, salts of dicarboxylic acids, and aluminosilicate salts. Suitable divalent sequestering gents are disclosed in British Patent Application No. 2 094 826 A.

### B. Alkalis or inorganic electrolytes

The composition may contain from about 1 to about 50 weight percent, preferably from about 5 to about 30 weight percent, based on the composition of one or more alkali metal salts of the following compounds as the alkalis or inorganic electrolytes: silicates, carbonates and sulfates as well as organic alkalis such as triethanolamine, diethanolamine, monoethanolamine and triisopropanolamine.

### Antiredeposition Agents

The composition may contain from about 0.1 to about 5 weight percent of one or more of the following compounds as antiredeposition agents: polyethylene glycol, polyvinyl alcohol, polyvinylpyrrolidone and carboxymethylcellulose.

Among them, a combination of carboxymethyl-cellulose and/or polyethylene glycol with the cellulase composition of the present invention provides for an especially useful dirt removing composition.

### Bleaching Agents

The use of the cellulase of the present invention in combination with a bleaching agent such as potassium monopersulfate, sodium percarbonate, sodium perborate, sodium sulfate/hydrogen peroxide adduct and sodium chloride/hydrogen peroxide adduct or/and a photo-sensitive bleaching dye such as zinc or aluminum salt of sulfonated phthalocyanine further improves the detergenting effects. Similarly, bleaching agents and bleach catalysts as described in EP 684 304 may be used.

### Bluing Agents and Fluorescent Dyes

Various bluing agents and fluorescent dyes may be incorporated in the composition, if necessary. Suitable bluing agents and fluorescent dyes are disclosed in British Patent Application No. 2 094 826 A.

### Caking Inhibitors

The following caking inhibitors may be incorporated in the powdery detergent: p-toluenesulfonic acid salts, xylenesulfonic acid salts, acetic acid salts, sulfosuccinic acid salts, talc, finely pulverized silica, amorphous silicas, clay, calcium silicate (such as Micro-Cell of Johns Manville Co.), calcium carbonate and magnesium oxide.

### Masking Agents for Factors inhibiting the Cellulase Activity

The cellulase composition of this invention are deactivated in some cases in the presence of copper, zinc, chromium, mercury, lead, manganese or silver ions or their compounds. Various metal chelating agents and metal-precipitating agents are effective against these inhibitors. They include, for example, divalent metal ion sequestering agents as listed in the above item with reference to optional additives as well as magnesium silicate and magnesium sulfate.

Cellobiose, glucose and gluconolactone act sometimes as inhibitors, It is preferred to avoid the co-presence of these saccharides with the cellulase as far as possible. In case the co-presence in unavoidable, it is necessary to avoid the direct contact of the saccharides with the cellulase by, for example, coating them.

Long-chain-fatty acid salts and cationic surfactants act as the inhibitors in some cases. However, the co-presence of these substances with the cellulase is allowable if the direct contact of them is prevented by some means such as tableting or coating.

The above-mentioned masking agents and methods may be employed, if necessary, in the present invention.

### Cellulase-Activators

The activators may vary depending on the specific cellulase. In the presence of proteins, cobalt and its salts, magnesium and its salts, and calcium and its salts, potassium and its salts, sodium and its salts or monosaccharides such as mannose and xylose, many cellulases are activated and their deterging powers are improved remarkably.

### Antioxidants

The antioxidants include, for example, tert-butyl-hydroxytoluene, 4,4'-butylidenebis(6-tert-butyl-3-methylphenol), 2,2'-butylidenebis(6-tert-butyl-4-methylphenol), monostyrenated cresol, distyrenated cresol, monostyrenated phenol, distyrenated phenol and 1,1-bis(4-hydroxy-phenyl)cyclohexane.

### Solubilizers

The solubilizers include, for example, lower alcohols such as ethanol, benzenesulfonate salts, lower alkylbenzenesulfonate salts such as p-toluenesulfonate salts, glycols such as propylene glycol, acetylbenzene-sulfonate salts, acetamides, pyridinedicarboxylic acid amides, benzoate salts and urea.

The detergent composition of the present invention can be used in a broad pH range from acidic to alkaline pH. In a preferred embodiment, the detergent composition of the present invention can be used in mildly acidic, neutral or alkaline detergent wash media having a pH of from above 5 to no more than about 12.

Aside from the above ingredients, perfumes, buffers, preservatives, dyes and the like can be used, if desired, with the detergent compositions of this invention. Such components are conventionally employed in amounts heretofore used in the art.

When a detergent base used in the present invention is in the form of a powder, it may be one which is prepared by any known preparation methods including a spray-drying method and a granulation method. The detergent base obtained particularly by the spray-drying method, agglomeration method, dry mixing method or non-tower route methods are preferred. The detergent base obtained by the spray-drying method is not restricted with respect to preparation conditions. The detergent base obtained by the spray-drying method is hollow granules which are obtained by spraying an aqueous slurry of heat-resistant ingredients, such as surface active agents and builders, into a hot space. After the spray-drying, perfumes, enzymes, bleaching agents, inorganic alkaline builders may be added. With a highly dense, granular detergent base obtained such as by the spray-drying-granulation or agglomeration method, various ingredients may also be added after the preparation of the base.

When the detergent base is a liquid, it may be either a homogeneous solution or an inhomogeneous dispersion. For removing the decomposition of carboxymethylcellulose by the cellulase in the detergent, it is desirable that carboxymethylcellulose is granulated or coated before the incorporation in the composition.

The detergent compositions of this invention may be incubated with cellulose containing fabric, for example soiled fabrics, in industrial and household uses at temperatures, reaction times and liquor ratios conventionally employed in these environments. The incubation conditions, i.e., the conditions effective for treating cellulose containing fabrics with detergent compositions according to the present invention, will be readily ascertainable by those of skill in the art. Accordingly, the appropriate conditions effective for treatment with the present detergents will correspond to those using similar detergent compositions which include known cellulases.

Detergents according to the present invention may additionally be formulated as a pre-wash in the appropriate solution at an intermediate pH where sufficient activity exists to provide desired improvements softening, depilling, pilling prevention, surface fiber removal or cleaning. When the detergent composition is a pre-soak (e.g., pre-wash or pre-treatment) composition, either as a liquid, spray, gel or paste composition, the cellulase enzyme is generally employed from about 0.0001 to about 1 weight percent based on the total weight of the pre-soak or pre-treatment composition. In such compositions, a surfactant may optionally be employed and when employed, is generally present at a concentration of from about 0.005 to about 20 weight percent based on the total weight of the pre-soak, The remainder of the composition comprises conventional components used in the pre-soak. i.e.. diluent, buffers, other enzymes (proteases), and the like at their conventional concentrations.

It is contemplated that compositions comprising cellulase enzymes described herein can be used in home use as a stand alone composition suitable for restoring color to faded fabrics (see, for example, U.S. Patent No. 4,738,682) as well as used in a spot-remover and for depilling and antipilling (pilling prevention).

The use of the cellulase according to the invention may be particularly effective in feed additives and in the processing of pulp and paper. These additional industrial applications are described in, for example, PCT Publication No. WO 95/16360 and Finnish Granted Patent No. 87372, respectively.

In order to further illustrate the present invention and advantages thereof, the following specific examples are given with the understanding that they are being offered to illustrate the present invention and should not be construed in any way as limiting its scope.

### EXAMPLE

Genomic DNA was prepared for several different microorganisms for the purpose of undertaking a PCR reaction to determine whether EGIII like enzymes are encoded by the DNA for a particular organism.

Genomic DNA is obtained from *Acremonium brachypenium* deposit no. CBS 866.73; *Chaetomium brasillience* deposit no. CBS 140.50; *Chaetomium vitellium* deposit no. CBS 250.85; *Emericella desertoru* deposit no. CBS 653.73; *Fusarium equiseti* deposit no. CBS 185.34; *Gliocladium roseum* deposit no. CBS 443.65; *Humicola grisea var. thermoidia* deposit no. CBS 225.63; *Myceliopthora thermophila* deposit no. ATCC 48102-48104; *Penicillium notatum* deposit no. ATCC 9178, 9179; *and Phanerochaete chrysosporium* deposit no. ATCC 28326 and isolated according to standard methods.

PCR was performed on a standard PCR machine such as the PCT-150 MicroCycler from MJ Research Inc. under the following conditions:
1) 1 minute at 98°C for 1 cycle;
2) 1 minute at 94°C,
   90 seconds at 40°C,
   1 minute at 72°C
3) repeat step 2 for 30 cycles
4) 7 minutes at 72°C for 1 cycle
5) lower temperature to 15°C for storage and further analysis.

The following DNA primers were constructed for use in amplification of EGIII like genes from the libraries constructed from the various microorganisms. All symbols used herein for protein and DNA sequences correspond to IUPAC IUB Biochemical Nomenclature Commission codes.

**BOX1: primers coding for (N/Q)NLWG**

| | |
|---|---|
| forward primer | FRG001: AAY AAY YTN TGG GG |
| forward primer | FRG002: CAR AAY YTN TGG GG |

**BOX1': primers coding for NNN(F/L/Y/I/L/N/K)WG**

| | |
|---|---|
| forward primer | FRG010: AAY AAY AAY HWI TGG GG |

**BOX2: primers coding for ELMIW**

| | |
|---|---|
| forward primer | FRG003: GAR YTN ATG ATH TGG |
| reversed primer | FRG004: CCA DAT CAT NAR YTC |

**BOX2': primers coding for YELMIW**

| | |
|---|---|
| forward primer | FRG011: TAY GAR YTI ATG ATH TGG |
| reversed primer | FRG012: CCA DAT CAT IAR YTC RTA |

**BOX3: primers coding for GTE(P/C)FT**

| | |
|---|---|
| reversed primer | FRG005: GTR AAN GGY TCR GTR CC |
| reversed primer | FRG006: GTR AAN GGY TCR GTY CC |
| reversed primer | FRG007: GTR AAN GGY TCY GTR CC |
| reversed primer | FRG008: GTR AAN GGY TCY GTY CC |
| reversed primer | FRG009: GTR AAR CAY TCN GTN CC |

PCR conditions for PWO polymerase (Boehringer Mannheim, Cat # 1644-947) comprise a 100 microliter solution made of 10 microliter of 10X reaction buffer (10X reaction buffer comprising 100mM Tris HCl, pH 8-8.5; 250 mM KCl; 50 mM (NH₄)₂SO₄; 20 mM MgSO₄); 0.2 mM each of dATP, dTTP, dGTP, dCTP (final concentration), 1 microliter of 100 nanogram/microliter genomic DNA, 1 microliter of PWO at 1 unit per microliter, 500 mM primers (final concentration) and water to 100 microliters. The solution is overlaid with mineral oil.

The PCR strategy was as follows: forward primers for BOX1 and BOX1' were combined with reversed primers from BOX3 in a mixture with the desired genomic DNA sample and run on a gel to obtain fragments in the 400-1000 base pair range. The obtained fragments were then pooled and the pool split into two approximately equal portions. The first pool was combined with the forward primers from BOX1 and BOX1' along with the reversed primer from BOX2. The second pool was combined with the forward primer from BOX2 along with the reversed primers from BOX3. Fragments having the approximate size relative to an EGIII like cellulase considering the location of the primers within the gene, in this case corresponding to those between 250-500 base pairs, were isolated and sequenced. Partial sequences for EGIII like cellulase genes are provided in Fig. 3.

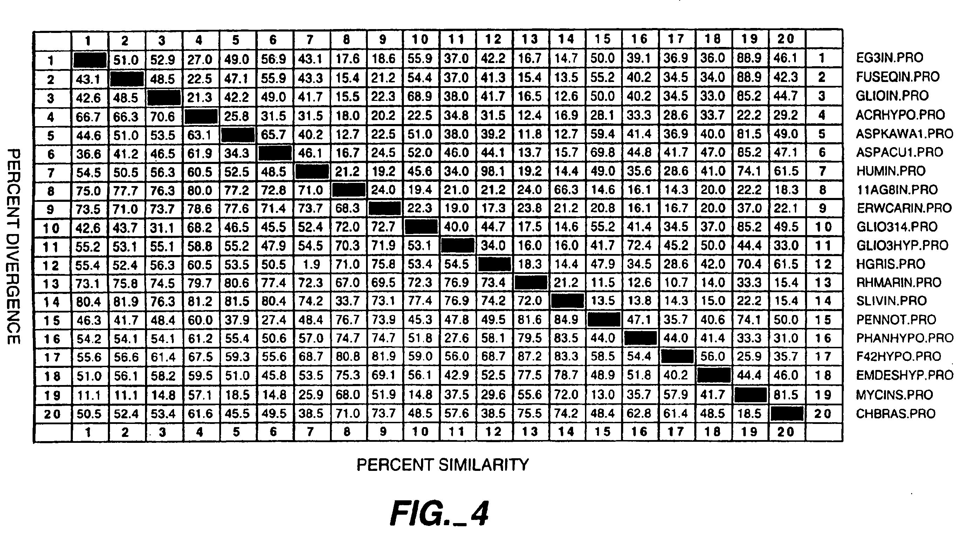

The isolated and partially sequenced DNA and the corresponding amino acid sequences (of approximately 100 residues) were analyzed to determine their relationship to EGIII. The results of this sequence alignment are shown in Fig. 3. As shown in Fig. 3, significant sequence homology exists between the peptides encoded by the obtained DNA fragments and corresponding peptide sequences from EGIII. Due to this homology, it was concluded by Applicants that the nature of the numerous conserved residues identify the fragment as corresponding to a gene encoding a cellulase. Moreover, the high homology and strong conservation of residues corresponding to peptides (a), (b), (c) and/or (d), as in EGIII, identify the genes as coding an EGIII like enzyme from each of the organisms. Fig. 4 illustrates the percent similarity of the protein fragments sequenced.

From the sequenced fragments, it was possible to use the RAGE technique (rapid amplification of genomic ends) to rapidly obtain the sequence of the full length gene. Full length genes were obtained and are provided with several additional EGIII-like cellulase sequences in Fig. 6. As shown in Fig. 6, full length genes isolated from *Hypocrea schweinitzii, Aspergillus aculeatus, Aspergillus kawachii (1), Aspergillus kawachii (2), Aspergillus oryzae, Humicola grisea, Humicola insolens, Chaetomium brasilliense, Fusarium equiseti, Fusarium javanicum (1). Fusarium javanicum (2), Gliocladium roseum (1), Gliocladium roseum (2), Gliocladium roseum (3), Gliogladium roseum (4), Memnoniella echinata, Actinomycete 11AG8, Streptomyces lividans CelB, Rhodothermus marinus, Emericella desertoru,* and *Erwinia carotovara* all comprise significant homology EGIII from *Trichoderma reesei.*

## Claims

1. A method for obtaining a gene encoding an EGIII like cellulase comprising the steps of:
(i) preparing genomic DNA from an organism of interest;
(ii) preparing a DNA primer encoding an amino acid string selected from the group consisting of one or more of the following:
(a) Asn-Asn-(Leu/Phe/Lys/lle)-Trp-Gly
(b) Glu-(Leu/Phe/lle)-Met-Ile-Trp
(c) Gly-Thr-Glu-Pro-Phe-Thr;
(d) (Ser/Tyr/Cys/Trp/Thr/Asn/Lys/Arg)-(Val/Pro)-(Lys/Ala)-(Ser/Ala)-(Tyr/Phe); and
(e) Lys-Asn-Phe-Phe-Asn-Tyr;
(iii) amplifying the genomic DNA from step (i) by the polymerase chain reaction (PCR) using DNA primers encoding amino acid strings (a) and/or (d) and (c) and/or (e) under conditions suitable for producing one or more gene fragments from said genomic DNA;
(iv) selecting and pooling the gene fragments amplified in step (iii) and reamplifying using DNA primers encoding amino acid strings (a) and/or (d) plus (b), or using DNA primers encoding amino acid strings (c) and/or (e) plus (b) to produce one or more further gene fragments; and
(v) isolating from said genomic DNA all or part of the gene corresponding to one or more the further gene fragments produced in step (iv).

2. The method according to claim 1, wherein said step (iii) further comprises labeling said DNA primer and performing hybridization between said labeled DNA primer and said genomic DNA and detecting said hybridized genomic DNA which encodes all or part of an EGIII like cellulase.

3. The method according to claim 1 or claim 2, wherein the gene fragments are selected by running the amplification products on a polyacrylamide gel and selecting gene fragments of a suitable size range,

4. The method according to any one of the preceding claims, wherein gene fragments in the size range of 400-1000 base pairs are selected in step (iii).

5. The method according to any one of the preceding claims, wherein gene fragments in the size range of 250-500 base pairs are selected in step (iv).

6. The method according to any one of the preceding claims, further comprising sequencing one or more of the gene fragments produced in step (iv).

7. The method according to claim 1, wherein said genomic DNA is obtained from a bacteria, fungus or Actinomycete.

8. The method according to claim 7, wherein said fungus is a filamentous fungus.

9. The method according to claim 8, wherein said filamentous fungus belongs to Euascomycete.

10. The method according to claim 9, wherein said Euascomycete belongs to Plectomycete.

11. The method according to claim 9, wherein said Euascomycete belongs to Diaporthales, Halosphaeriales, Microascales, Ophiostomatales, Phyllachorales, Sordariales or Xylariales.

12. The method according to claim 9, wherein said Eusacomycete belongs to Hypocreales comprising Clavicipitaceae, Melanosporaceae, Nectriaceae, Niessliaceae or Mitosporic Hypocreales.

13. The method according to claim 9, wherein said Euascomycete belongs to Hypocreaceae, wherein said Hypocreaceae does not comprise Trichoderma.

14. The method according to claim 9, wherein said Euascomycete is *Gliocladium spp., Fusarium spp., Acremonium spp., Myceliophtora spp., Verticillium spp., Myrothecium spp.,* or *Penicillium spp.*

15. The method according to claim 9, wherein said Euascomycete is an *Aspergillus* comprising *A. aeneus, A. anthodesmis, A. aureofulgens, A. aureolatus, A. avenaceus, A. awamorii, A. bisporus, A. brunneouniseriatus, A. campestris, A. caesiellus, A. candidus, A. carbonarius, A. cameus, A. cervinus, A. clavatoflavus, A. clavatoanicus, A. clavatus, A. conicus, A. conjunctus, A. crustosus, A deffectus, A. dimorphicus, A. eburneocremeus,A, egyptiacus,A. ellipticus,A. elongatus,A. ficuum,A. flaschentraegeri, A, flavus, a. fumigatus, A. giganteus, A. glaucus, A, gorakhpurensis, A. gracilis, A. iizuke, A. itaconicus, A. japonicus, A. kambarensis, A. kanagawaensis, A. lanosus, A. leporis, A. longivesica, A. mellinus, A. multicolor, A. niger, A. nomius, A. nutans, A. ochraceus, A. oryzae, A. pallidus, A. panamensis, A. parasiticus, A. parvulus, A. penicillioides, A. phialisepticus, A. phoenicis, A. proliferans, A.pulvinus, A.puniceus, A.raperi, A.recurvatus, A.restrictus, A.shirousami*, *A.sojae, A.sparsus, A.subolivaceus, A. subsessilis, A. tamarri, A. terreus, A. terricola, A. thomii, A. tubingensis, A. unguis, A.unilateralis, A.ustus, A.versicolor, A.wentii, A.xerophilus, A.zonatus, A.sp.*

16. A method which comprises, having carried out the method of any one of the preceding claims and obtained a gene encoding an EGIII like cellulase, the further steps of:
(a) transforming a host cell with the gene encoding an EGIII-like cellulase;
(b) culturing the host cell comprising the gene in a suitable culture medium under suitable conditions to produce cellulase;
(c) obtaining said produced cellulase; and optionally
(d) purifying said cellulase to provide a purified cellulase product.

## Patentansprüche

1. Verfahren zum Erhalt eines Gens, das eine EGIII-ähnliche Cellulase codiert, umfassend die folgenden Schritte:
(i) Herstellen von genomischer DNA aus einem Organismus von Interesse;
(ii) Herstellen eines DNA-Primers, der einen Aminosäurestrang codiert, gewählt aus der Gruppe, bestehend aus einem oder mehreren der Folgenden:
(a) Asn-Asn-(Leu/Phe/Lys/Ile)-Trp-Gly
(b) Glu-(Leu/Phe/Ile)-Met-Ile-Trp
(c) Gly-Thr-Glu-Pro-Phe-Thr;
(d) (Ser/Tyr/Cys/Trp/Thr/Asn/Lys/Arg)-(Val/Pro)-(Lys/Ala)-(Ser/Ala)-(Tyr/Phe); und
(e) Lys-Asn-Phe-Phe-Asn-Tyr;
(iii) Amplifizieren der genomischen DNA aus Schritt (i) durch die Polymerase-Kettenreaktion (PCR) unter Verwendung von DNA-Primern, codierend die Aminosäurestränge (a) und/oder (d) und (c) und/oder (e), unter Bedingungen, die zur Herstellung eines oder mehrerer Genfragmente aus der genomischen DNA geeignet sind;
(iv) Auswählen und Vereinigen der in Schritt (iii) amplifizierten Genfragmente und erneutes Amplifizieren unter Anwendung von DNA-Primern, die die Aminosäurestränge (a) und/oder (d) plus (b) codieren, oder unter Anwendung von DNA-Primern, die die Aminosäurestränge (c) und/oder (e) plus (b) codieren, um ein oder mehrere weitere Genfragmente herzustellen;
(v) Isolieren von der genomischen DNA der Gesamtheit oder eines Teils des Gens, welches einem oder mehreren der weiteren Genfragmente, die in Schritt (iv) hergestellt wurden, entspricht.

2. Verfahren gemäß Anspruch 1, wobei der Schritt (iii) ferner das Markieren des DNA-Primers und das Durchführen einer Hybridisierung zwischen dem markierten DNA-Primer und der genomischen DNA und das Detektieren der hybridisierten genomischen DNA, welche die Gesamtheit oder einen Teil einer EGIII-ähnlichen Cellulase codiert, umfasst.

3. Verfahren gemäß Anspruch 1 oder Anspruch 2, wobei die Genfragmente gewählt werden, indem die Amplifikationsprodukte auf einem Polyacrylamidgel laufen gelassen werden und Genfragmente eines geeigneten Größenbereichs gewählt werden.

4. Verfahren gemäß mindestens einem der vorstehenden Ansprüche, wobei Genfragmente im Größenbereich von 400 - 1 000 Basenpaare in Schritt (iii) gewählt werden.

5. Verfahren gemäß mindestens einem der vorhergehenden Ansprüche, wobei Genfragmente im Größenbereich von 250 - 500 Basenpaaren im Schritt (iv) gewählt werden.

6. Verfahren gemäß mindestens einem der vorstehenden Ansprüche, ferner das Sequenzieren von einem oder mehreren der Genfragmente, die in Schritt (iv) erzeugt wurden, umfassend.

7. Verfahren gemäß Anspruch 1, wobei die genomische DNA aus einem Bakterium, Pilz oder Actinomyceten erhalten wird.

8. Verfahren gemäß Anspruch 7, wobei der Pilz ein filamentöser Pilz ist.

9. Verfahren gemäß Anspruch 8, wobei der filamentöse Pilz zu den Euascomyceten gehört.

10. Verfahren gemäß Anspruch 9, wobei die Euascomycete zu Plectomycete gehört.

11. Verfahren gemäß Anspruch 9, wobei die Euascomycete zu Diaporthales, Halosphaeriales, Microascales, Ophiostomatales, Phyllachorales, Sordariales oder Xylariales gehört.

12. Verfahren gemäß Anspruch 9, wobei die Euascomycete zu Hypocreales, umfassend Clavicipitaceae, Melanosporaceae, Nectriaceae, Niessliaceae oder zum Mitosporus Hypocreales, gehört.

13. Verfahren gemäß Anspruch 9, wobei die Euascomycete zu Hypocreaceae gehört, wobei die Hypocreaceae nicht Trichoderma umfasst.

14. Verfahren gemäß Anspruch 9, wo die Euascomycete *Gliocladium spp., Fusarium spp., Acremonium spp., Myceliophtora spp., Verticillium spp., Myrothecium spp.,* oder *Penicillium spp.* ist.

15. *Verfahren gemäß Anspruch 9, wobei die Euascomycete ein Aspergillus, umfassend A. aeneus, A. anthodesmis, A. aureofulgens, A. aureolatus, A. avenaceus, A. awamorii, A. bisporus, A. brunneouniseriatus, A. campestris, A. caesiellus, A. candidus, A. carbonarius, A. cameus, A. cervinus, A. clavatroflavus, A. clavatoanicus, A. clavatus, A. conicus, A. conjunctus, A. crustosus, A. deflectus, A. dimorphicus, A. eburneocremeus, A. egyptiacus, A. ellipticus, A. elongatus, A. ficuum, A. flaschentraegeri, A. flavus, A. fumigatus, A. giganteus, A. glaucus, A. gorakhpurensis, A. gracilis, A. iizuke, A. itaconicus, A. japonicus, A. kambarensis, A. kanagawaensis, A. lanosus, A. leporis, A. longivesica, A. mellinus, A. multicolor, A. niger, A. nomius, A. nutans, A. ochraceus, A. oryzae, A. pallidus, A. panamensis, A. parasiticus, A. parvulus, A. penicillioides, A. phialisepticus, A. phoenicis, A. proliferans, A. pulvinus, A. puniceus, A. raperi, A. recurvatus, A. restrictus, A. shirousami, A. sojae, A. sparsus, A. subolivaceus, A. subsessilis, A. tarnarii, A. terreus, A. terricola, A. thomii, A. tubingensis, A. unguis, A. unilateralis, A. ustus, A. versicolor, A. wentii, A. xerophilus, A. zonatus, A. sp.,* ist.

16. Verfahren, welches die folgenden weiteren Schritt umfasst, nachdem das Verfahren gemäß mindestens einem der vorstehenden Ansprüche durchgeführt worden ist und ein Gen erhalten wurde, das eine EGIII-ähnliche Cellulase codiert:
(a) Transformieren einer Wirtszelle mit dem Gen, das EGIII-ähnliche Cellulase codiert;
(b) Kultivieren der das Gen umfassenden Wirtszelle in einem geeigneten Kulturmedium unter stabilen Bedingungen zur Herstellung von Cellulase;
(c) Erhalten der hergestellten Cellulase; und ggf.
(d) Reinigen der Cellulase, um ein gereinigtes Cellulaseprodukt bereitzustellen.

## Revendications

1. Procédé pour obtenir un gène codant pour une cellulase de type EGIII comprenant les étapes de :
(i) préparation d'ADN génomique à partir d'un organisme d'intérêt ;
(ii) préparation d'une amorce d'ADN codant pour une chaîne d'acides aminés sélectionnée dans le groupe comprenant un ou plusieurs des éléments suivants :
(a) Asn-Asn-(Leu/Phe/Lys/Ile)-Trp-Gly
(b) Glu-(Leu/Phe/Ile)-Met-Ile-Trp
(c) Gly-Thr-Glu-Pro-Phe-Thr ;
(d) (Ser/Tyr/Cys/Trp/Thr/Asn/Lys/Arg)-(Val/Pro)-(Lys/Ala)-(Ser/Ala)-(Tyr/Phe) ; et
(e) Lys-Asn-Phe-Phe-Asn-Tyr ;
(iii) amplification de l'ADN génomique à partir de l'étape (i) par l'amplification en chaîne par polymérase (ACP) en utilisant des amorces d'ADN codant pour des chaînes d'acides aminés (a) et/ou (d) et (c) et/ou (e), dans des conditions appropriées pour produire un ou plusieurs fragments de gène à partir dudit ADN génomique ;
(iv) sélection et groupement des fragments de gène amplifiés à l'étape (iii) et réamplification en utilisant des amorces d'ADN codant pour des chaînes d'acides aminés (a) et/ou (d) plus (b), ou en utilisant des amorces d'ADN codant pour des chaînes d'acides aminés (c) et/ou (e) plus (b) pour produire un ou plusieurs autres fragments de gène ; et
(v) isolation dudit ADN génomique de la totalité ou d'une partie du gène correspondant à un ou plusieurs autres fragments de gène produits à l'étape (iv).

2. Procédé selon la revendication 1, dans lequel ladite étape (iii) comprend, en outre, le marquage de ladite amorce d'ADN et la réalisation d'une hybridation entre ladite amorce d'ADN marquée et ledit ADN génomique et la détection dudit ADN génomique hybridé qui code pour la totalité ou une partie d'une cellulase de type EGIII.

3. Procédé selon la revendication 1 ou la revendication 2, dans lequel les fragments de gène sont sélectionnés en déposant les produits d'amplification sur un gel de polyacrylamide et en sélectionnant des fragments de gène d'une plage de tailles appropriée.

4. Procédé selon l'une quelconque des revendications précédentes, dans lequel des fragments de gène dans la plage de taille de 400 à 1000 paires de bases sont sélectionnés à l'étape (iii).

5. Procédé selon l'une quelconque des revendications précédentes, dans lequel des fragments de gène dans la plage de taille de 250 à 500 paires de bases sont sélectionnés à l'étape (iv).

6. Procédé selon l'une quelconque des revendications précédentes, comprenant, en outre, le séquençage d'un ou de plusieurs des fragments de gène produits à l'étape (iv).

7. Procédé selon la revendication 1, dans lequel ledit ADN génomique est obtenu à partir d'une bactérie, d'un champignon ou d'un Actinomycète.

8. Procédé selon la revendication 7, dans lequel ledit champignon est un champignon filamenteux.

9. Procédé selon la revendication 8, dans lequel ledit champignon filamenteux appartient à Euascomycète.

10. Procédé selon la revendication 9, dans lequel ledit Euascomycète appartient à Plectomycète.

11. Procédé selon la revendication 9, dans lequel ledit Euascomycète appartient à Diaporthales, Halosphaeriales, Microascales, Ophiostomatales, Phyllachorales, Sordariales ou Xylariales

12. Procédé selon la revendication 9, dans lequel ledit Euascomycète appartient à Hypocreates comprenant Clavicipitaceae, Melanosporaceae, Nectriaceae, Niessliaceae ou Hypocreales Mitosporique.

13. Procédé selon la revendication 9, dans lequel ledit Euascomycète appartient à Hypocreaceae, dans lequel ledit Hypocreaceae ne comprend pas Trichoderma.

14. Procédé selon la revendication 9, dans lequel ledit Euascomycète est un *Gliocladium spp.,* un *Fusarium spp.,* un *Acremonium spp.,* un *Myceliophtora spp.,* un *Verticillium spp.,* un *Myrothecium spp.,* ou un *Penicillium spp.*

15. Procédé selon la revendication 9, dans lequel ledit Euascomycète est un *Aspergillus* comprenant *A. aeneus, A. anthodesmis, A. aureofulgens, A. aureolatus, A. avenaceus, A. awamonii, A. bisporus, A. brunneouniseriatus, A. campestris, A. caesiellus, A. candidus, A. carbonarius, A. cameus, A. cervinus, A. clavatoflavus, A. clavatoanicus, A. clavatus, A. conicus, A. conjunctus, A. crustosus, A. deflectus, A. dimorphicus, A. eburneocremeus, A. egyptiacus, A. ellipticus, A. elongatus, A. ficuum, A. flaschentraegeri, A. flavus, A. fumigatus, A. giganteus, A. glaucus, A. gorakhpurensis, A. gracilis, A. iizuke, A. itaconicus, A. japonicus, A. kambarensis, A. kanagawaensis, A. lanosus, A. leporis, A. longivesica, A. mellinus, A. multicolor, A. niger, A. nomius, A. nutans, A. ochraceus, A. oryzae, A. pallidus, A. panamensis, A. parasiticus, A. parvulus, A. penicillioides, A. phialisepticus, A. phoenicis, A. proliferans, A. pulvinus, A. puniceus, A. raperi, A. recurvatus, A. restrictus, A. shirousami, A. sojae, A. sparsus, A. subolivaceus, A. subsessilis, A. tamarii, A. terreus, A. terricola, A. thornii, A. tubingensis, A. unguis, A. unilateralis, A. ustus, A. versicolor, A. wentii, A. xerophilus, A. zonatus, A. sp.*

16. Procédé qui comprend, après avoir réalisé le procédé de l'une quelconque des revendications précédentes et obtenu un gène codant pour une cellulase de type EGIII, les étapes supplémentaires de :
(a) transformation d'une cellule hôte par le gène codant pour une cellulase de type EGIII ;
(b) mise en culture de la cellule hôte comprenant le gène dans un milieu de culture approprié dans des conditions appropriées pour produire une cellulase ;
(c) obtention de ladite cellulase produite ; et facultativement
(d) purification de ladite cellulase pour fournir un produit de cellulase purifié.


### SEQUENCE LISTING

<110> Genencor International, Inc.
<120> Novel EGIII-Like Enzymes, DNA Encoding Such Enzymes and Methods for Producing Such Enzymes
<130> GC516-2-PCT
<140> U.S.Serial No. 08/991,720
   <141> 1997-12-16
<150> 41
<160> FastSEQ for Windows Version 3.0
<210> 1
   <211> 5
   <212> PRT
   <213> Artificial Sequence
<220>
   <223> Synthetic
<400> 1
   Asn Asn Xaa Trp Gly
    1               5
<210> 2
   <211> 5
   <212> PRT
   <213> Artificial Sequence
<220>
   <223> Synthetic
<400> 2
   Glu Xaa Met Ile Trp
   1 5
<210> 3
   <211> 6
   <212> PRT
   <213> Artificial Sequence
<220>
   <223> Synthetic
<400> 3
   Gly Thr Glu Pro Phe Thr
   1 5
<210> 4
   <211> 5
   <212> PRT
   <213> Artificial Sequence
<220>
   <223> Synthetic
<400> 4
   Ser Val Lys Ser Tyr
   1 5
<210> 5
   <211> 6
   <212> PRT
   <213> Artificial Sequence
<220>
   <223> Synthetic
<400> 5
   Lys Asn Phe Phe Asn Tyr
   1 5
<210> 6
   <211> 702
   <212> DNA
   <213> T. reesei
<400> 6
   atgaagttcc ttcaagtcct ccctgccctc ataccggccg ccctggccca aaccagctgt 60
   gaccagtggg caaccttcac tggcaacggc tacacagtca gcaacaacct ttggggagca 120
   tcagccggct ctggatttgg ctgcgtgacg gcggtatcgc tcagcggcgg ggcctcctgg 180
   cacgcagact ggcagtggtc cggcggccag aacaacgtca agtcgtacca gaactctcag 240
   attgccattc cccagaagag gaccgtcaac agcatcagca gcatgcccac cactgccagc 300
   tggagctaca gcgggagcaa catccgcgct aatgttgcgt atgacttgtt caccgcagcc 360
   aacccgaatc atgtcacgta ctcgggagac tacgaactca tgatctggct tggcaaatac 420
   ggcgatattg ggccgattgg gtcctcacag ggaacagtca acgtcggtgg ccagagctgg 480
   acgctctact atggctacaa cggagccatg caagtctatt cctttgtggc ccagaccaac 540
   actaccaact acagcggaga tgtcaagaac ttcttcaatt atctccgaga caataaagga 600
   tacaacgctg caggccaata tgttcttagc taccaatttg gtaccgagcc cttcacgggc 660
   agtggaactc tgaacgtcgc atcctggacc gcatctatca ac 702
<210> 7
   <211> 232
   <212> PRT
   <213> T. reesei
<400> 7
   Met Lys Phe Leu Gln Val Leu Pro Ala Leu Ile Pro Ala Ala Leu Ala
   1 5 10 15
   Gln Thr Ser Cys Asp Gln Trp Ala Thr Phe Thr Gly Asn Gly Tyr Thr
   20 25 30
   Val Ser Asn Asn Leu Trp Gly Ala Ser Ala Gly Ser Gly Phe Gly Cys
   35 40 45
   Val Thr Ala Val Ser Leu Ser Gly Gly Ala His Ala Asp Trp Gln Trp
   50 55 60
   Ser Gly Gly Gln Asn Asn Val Lys Ser Tyr Gln Asn Ser Gln Ile Ala
   65 70 75 80
   Ile Pro Gln Lys Arg Thr Val Asn Ser Ile Ser Ser Met Pro Thr Thr
   85 90 95
   Ala Ser Trp Ser Tyr Ser Gly Ser Asn Ile Arg Ala Asn Val Ala Tyr
   100 105 110
   Asp Leu Phe Thr Ala Ala Asn Pro Asn His Val Thr Tyr Ser Gly Asp
   115 120 125
   Tyr Glu Leu Met Ile Trp Leu Gly Lys Tyr Gly Asp Ile Gly Pro Ile
   130 135 140
   Gly Ser Ser Gln Gly Thr Val Asn Val Gly Gly Gln Ser Trp Thr Leu
   145 150 155 160
   Tyr Tyr Gly Tyr Asn Gly Ala Met Gln Val Tyr Ser Phe Val Ala Gln
   165 170 175
   Thr Asn Thr Thr Asn Tyr Ser Gly Asp Val Lys Asn Phe Phe Asn Tyr
   180 185 190
   Leu Arg Asp Asn Lys Gly Tyr Asn Ala Ala Gly Gln Tyr Val Leu Ser
   195 200 205
   Tyr Gln Phe Gly Thr Glu Pro Phe Thr Gly Ser Gly Thr Leu Asn Val
   210 215 220
   Ala Ser Trp Thr Ala Ser Ile Asn
   225 230
<210> 8
   <211> 234
   <212> PRT
   <213> T. reesei
<400> 8
   Met Lys Phe Leu Gln Val Leu Pro Ala Leu Ile Pro Ala Ala Leu Ala
    1               5                  10                  15
   Gln Thr Ser Cys Asp Gln Trp Ala Thr Phe Thr Gly Asn Gly Tyr Thr
               20                  25                  30
   Val Ser Asn Asn Leu Trp Gly Ala Ser Ala Gly Ser Gly Phe Gly Cys
           35                  40                  45
   Val Thr Ala Val Ser Leu Ser Gly Gly Ala Ser Trp His Ala Asp Trp
       50                  55                  60
   Gln Trp Ser Gly Gly Gln Asn Asn Val Lys Ser Tyr Gln Asn Ser Gln
   65                  70                  75                  80
   Ile Ala Ile Pro Gln Lys Arg Thr Val Asn Ser Ile Ser Ser Met Pro
                   85                  90                  95
   Thr Thr Ala Ser Trp Ser Tyr Ser Gly Ser Asn Ile Arg Ala Asn Val
               100                 105                 110
   Ala Tyr Asp Leu Phe Thr Ala Ala Asn Pro Asn His Val Thr Tyr Ser
           115                 120                 125
   Gly Asp Tyr Glu Leu Met Ile Trp Leu Gly Lys Tyr Gly Asp Ile Gly
       130                 135                 140
   Pro Ile Gly Ser Ser Gln Gly Thr Val Asn Val Gly Gly Gln Ser Trp
   145                 150                 155                 160
   Thr Leu Tyr Tyr Gly Tyr Asn Gly Ala Met Gln Val Tyr Ser Phe Val
                   165                 170                 175
   Ala Gln Thr Asn Thr Thr Asn Tyr Ser Gly Asp Val Lys Asn Phe Phe
               180                 185                 190
   Asn Tyr Leu Arg Asp Asn Lys Gly Tyr Asn Ala Ala Gly Gln Tyr Val
           195                 200                 205
   Leu Ser Tyr Gln Phe Gly Thr Glu Pro Phe Thr Gly Ser Gly Thr Leu
       210                 215                 220
   Asn Val Ala Ser Trp Thr Ala Ser Ile Asn
   225                 230
<210> 9
   <211> 234
   <212> PRT
   <213> H. schweinitzii
<400> 9
   Met Lys Phe Leu Gln Val Leu Pro Ala Ile Leu Pro Ala Ala Leu Ala
    1               5                  10                  15
   Gln Thr Ser Cys Asp Gln Tyr Ala Thr Phe Ser Gly Asn Gly Tyr Ile
               20                  25                  30
   Val Ser Asn Asn Leu Trp Gly Ala Ser Ala Gly Ser Gly Phe Gly Cys
           35                  40                  45
   Val Thr Ser Val Ser Leu Asn Gly Ala Ala Ser Trp His Ala Asp Trp
       50                  55                  60
   Gln Trp Ser Gly Gly Gln Asn Asn Val Lys Ser Tyr Gln Asn Val Gln
   65                  70                  75                  80
   Ile Asn Ile Pro Gln Lys Arg Thr Val Asn Ser Ile Gly Ser Met Pro
                   85                  90                  95
   Thr Thr Ala Ser Trp Ser Tyr Ser Gly Ser Asp Ile Arg Ala Asn Val
               100                 105                 110
   Ala Tyr Asp Leu Phe Thr Ala Ala Asn Pro Asn His Val Thr Tyr Ser
           115                 120                 125
   Gly Asp Tyr Glu Leu Met Ile Trp Leu Gly Lys Tyr Gly Asp Ile Gly
       130                 135                 140
   Pro Ile Gly Ser Ser Gln Gly Thr Val Asn Val Gly Gly Gln Thr Trp
   145                 150                 155                 160
   Thr Leu Tyr Tyr Gly Tyr Asn Gly Ala Met Gln Val Tyr Ser Phe Val
                   165                 170                 175
   Ala Gln Ser Asn Thr Thr Ser Tyr Ser Gly Asp Val Lys Asn Phe Phe
               180                 185                 190
   Asn Tyr Leu Arg Asp Asn Lys Gly Tyr Asn Ala Gly Gly Gln Tyr Val
           195                 200                 205
   Leu Ser Tyr Gln Phe Gly Thr Glu Pro Phe Thr Gly Ser Gly Thr Leu
       210                 215                 220
   Asn Val Ala Ser Trp Thr Ala Ser Ile Asn
   225                 230
<210> 10
   <211> 259
   <212> PRT
   <213> A. aculeatus
<400> 10
   Met Lys Ala Phe His Leu Leu Ala Ala Leu Ala Gly Ala Ala Val Ala
    1               5                  10                  15
   Gln Gln Ala Gln Leu Cys Asp Gln Tyr Ala Thr Tyr Thr Gly Gly Val
               20                  25                  30
   Tyr Thr Ile Asn Asn Asn Leu Trp Gly Lys Asp Ala Gly Ser Gly Ser
           35                  40                  45
   Gln Cys Thr Thr Val Asn Ser Ala Ser Ser Ala Gly Thr Ser Trp Ser
       50                  55                  60
   Thr Lys Trp Asn Trp Ser Gly Gly Glu Asn Ser Val Lys Ser Tyr Ala
   65                  70                  75                  80
   Asn Ser Gly Leu Thr Phe Asn Lys Lys Leu Val Ser Gln Ile Ser Gln
                   85                  90                  95
   Ile Pro Thr Thr Ala Arg Trp Ser Tyr Asp Asn Thr Gly Ile Arg Ala
               100                 105                 110
   Asp Val Ala Tyr Asp Leu Phe Thr Ala Ala Asp Ile Asn His Val Thr
           115                 120                 125
   Trp Ser Gly Asp Tyr Glu Leu Met Ile Trp Leu Ala Arg Tyr Gly Gly
       130                 135                 140
   Val Gln Pro Ile Gly Ser Gln Ile Ala Thr Ala Thr Val Asp Gly Gln
   145                 150                 155                 160
   Thr Trp Glu Leu Trp Tyr Gly Ala Asn Gly Ser Gln Lys Thr Tyr Ser
                   165                 170                 175
   Phe Val Ala Pro Thr Pro Ile Thr Ser Phe Gln Gly Asp Val Asn Asp
               180                 185                 190
   Phe Phe Lys Tyr Leu Thr Gln Asn His Gly Phe Pro Ala Ser Ser Gln
           195                 200                 205
   Tyr Leu Ile Thr Leu Gln Phe Gly Thr Glu Pro Phe Thr Gly Gly Pro
       210                 215                 220
   Ala Thr Leu Ser Val Ser Asn Trp Ser Ala Ser Val Gln Gln Ala Gly
   225                 230                 235                 240
   Phe Glu Pro Trp Gln Asn Gly Ala Gly Leu Ala Val Asn Ser Phe Ser
                   245                 250                 255
   Ser Thr Val
<210> 11
   <211> 239
   <212> PRT
   <213> A. kawachii
<400> 11
   Met Lys Leu Ser Met Thr Leu Ser Leu Phe Ala Ala Thr Ala Met Gly
    1               5                  10                  15
   Gln Thr Met Cys Ser Gln Tyr Asp Ser Ala Ser Ser Pro Pro Tyr Ser
               20                  25                  30
   Val Asn Gln Asn Leu Trp Gly Glu Tyr Gln Gly Thr Gly Ser Gln Cys
           35                  40                  45
   Val Tyr Val Asp Lys Leu Ser Ser Ser Gly Ala Ser Trp His Thr Lys
       50                  55                  60
   Trp Thr Trp Ser Gly Gly Glu Gly Thr Val Lys Ser Tyr Ser Asn Ser
   65                  70                  75                  80
   Gly Leu Thr Phe Asp Lys Lys Leu Val Ser Asp Val Ser Ser Ile Pro
                   85                  90                  95
   Thr Ser Val Thr Trp Ser Gln Asp Asp Thr Asn Val Gln Ala Asp Val
               100                 105                 110
   Ser Tyr Asp Leu Phe Thr Ala Ala Asn Ala Asp His Ala Thr Ser Ser
           115                 120                 125
   Gly Asp Tyr Glu Leu Met Ile Trp Leu Ala Arg Tyr Gly Ser Val Gln
       130                 135                 140
   Pro Ile Gly Lys Gln Ile Ala Thr Ala Thr Val Gly Gly Lys Ser Trp
   145                 150                 155                 160
   Glu Val Trp Tyr Gly Thr Ser Thr Gln Ala Gly Ala Glu Gln Lys Thr
                   165                 170                 175
   Tyr Ser Phe Val Ala Gly Ser Pro Ile Asn Ser Trp Ser Gly Asp Ile
               180                 185                 190
   Lys Asp Phe Phe Asn Tyr Leu Thr Gln Asn Gln Gly Phe Pro Ala Ser
           195                 200                 205
   Ser Gln His Leu Ile Thr Leu Gln Cys Gly Thr Glu Pro Phe Thr Gly
       210                 215                 220
   Gly Pro Ala Thr Phe Thr Val Asp Asn Trp Thr Ala Ser Val Asn
   225                 230                 235
<210> 12
   <211> 239
   <212> PRT
   <213> A. kawachii 2
<400> 12
   Met Lys Ala Phe His Leu Leu Ala Ala Leu Ser Gly Ala Ala Val Ala
    1               5                  10                  15
   Gln Gln Ala Gln Leu Cys Asp Gln Tyr Ala Thr Tyr Thr Gly Gly Val
               20                  25                  30
   Tyr Thr Ile Asn Asn Asn Leu Trp Gly Lys Asp Ala Gly Ser Gly Ser
           35                  40                  45
   Gln Cys Thr Thr Val Asn Ser Ala Ser Ser Ala Gly Thr Ser Trp Ser
       50                  55                  60
   Thr Lys Trp Asn Trp Ser Gly Gly Glu Asn Ser Val Lys Ser Tyr Ala
   65                  70                  75                  80
   Asn Ser Gly Leu Ser Phe Asn Lys Lys Leu Val Ser Gln Ile Ser His
                   85                  90                  95
   Ile Pro Thr Ala Ala Arg Trp Ser Tyr Asp Asn Thr Cys Ile Arg Arg
               100                 105                 110
   Gly Arg Ala Tyr Asp Leu Phe Thr Ala Ala Asp Ile Asn His Val Thr
           115                 120                 125
   Trp Ser Gly Asp Tyr Glu Leu Met Ile Trp Leu Ala Arg Tyr Gly Gly
       130                 135                 140
   Val Gln Pro Leu Gly Ser Gln Ile Ala Thr Ala Thr Val Glu Gly Gln
   145                 150                 155                 160
   Thr Trp Glu Leu Trp Tyr Gly Val Asn Gly Ala Gln Lys Thr Tyr Ser
                   165                 170                 175
   Phe Val Ala Ala Asn Pro Ile Thr Ser Phe Gln Gly Asp Ile Asn Asp
               180                 185                 190
   Phe Phe Lys Tyr Leu Thr Gln Asn His Gly Phe Pro Ala Ser Ser Gln
           195                 200                 205
   Tyr Leu Ile Ile Leu Ala Leu Gln Phe Gly Thr Glu Pro Phe Thr Gly
       210                 215                 220
   Gly Pro Ala Thr Leu Asn Val Ala Asp Trp Ser Ala Ser Val Gln
   225                 230                 235
<210> 13
   <211> 247
   <212> PRT
   <213> A. oryzae
<400> 13
   Met Lys Leu Ser Leu Ala Leu Ala Thr Leu Val Ala Thr Ala Phe Ser
    1               5                  10                  15
   Gln Glu Leu Cys Ala Gln Tyr Asp Ser Ala Ser Ser Pro Pro Tyr Ser
               20                  25                  30
   Val Asn Asn Asn Leu Trp Gly Gln Asp Ser Gly Thr Gly Phe Thr Ser
           35                  40                  45
   Gln Cys Val Tyr Val Asp Asn Leu Ser Ser Ser Gly Ala Ala Trp His
       50                  55                  60
   Thr Thr Trp Thr Trp Asn Gly Gly Glu Gly Ser Val Lys Ser Tyr Ser
   65                  70                  75                  80
   Asn Ser Ala Val Thr Phe Asp Lys Lys Leu Val Ser Asp Val Gln Ser
                   85                  90                  95
   Ile Pro Thr Asp Val Glu Trp Ser Gln Asp Phe Thr Asn Thr Asn Val
               100                 105                 110
   Asn Ala Asp Val Ala Tyr Asp Leu Phe Thr Ala Ala Asp Gln Asn His
           115                 120                 125
   Val Thr Tyr Ser Gly Asp Tyr Glu Leu Met Ile Trp Leu Ala Arg Tyr
       130                 135                 140
   Gly Thr Ile Gln Pro Ile Gly Thr Gln Ile Asp Thr Ala Thr Val Glu
   145                 150                 155                 160
   Gly His Thr Trp Glu Leu Trp Phe Thr Tyr Gly Thr Thr Ile Gln Ala
                   165                 170                 175
   Gly Ala Glu Gln Lys Thr Tyr Ser Phe Val Ser Ala Thr Pro Ile Asn
               180                 185                 190
   Thr Phe Gly Gly Asp Ile Lys Lys Phe Phe Asp Tyr Ile Thr Ser Lys
           195                 200                 205
   His Ser Phe Pro Ala Ser Ala Gln Tyr Leu Ile Asn Met Gln Phe Gly
       210                 215                 220
   Thr Glu Pro Phe Phe Thr Thr Gly Gly Pro Val Thr Phe Thr Val Pro
   225                 230                 235                 240
   Asn Trp Thr Ala Ser Val Asn
                   245
<210> 14
   <211> 254
   <212> PRT
   <213> H. grisei
<400> 14
   Met Leu Lys Ser Ala Leu Leu Leu Gly Ala Ala Ala Val Ser Val Gln
    1               5                  10                  15
   Ser Ala Ser Ile Pro Thr Ile Pro Ala Asn Leu Glu Pro Arg Gln Ile
               20                  25                  30
   Arg Ser Leu Cys Glu Leu Tyr Gly Tyr Trp Ser Gly Asn Gly Tyr Glu
           35                  40                  45
   Leu Leu Asn Asn Leu Trp Gly Lys Asp Thr Ala Thr Ser Gly Trp Gln
       50                  55                  60
   Cys Thr Tyr Leu Asp Gly Thr Asn Asn Gly Gly Ile Gln Trp Asn Thr
   65                  70                  75                  80
   Ala Trp Glu Trp Gln Gly Ala Pro Asp Asn Val Lys Asn Tyr Pro Tyr
                   85                  90                  95
   Val Gly Lys Gln Ile Gln Arg Gly Arg Lys Ile Ser Asp Ile Asn Ser
               100                 105                 110
   Met Arg Thr Ser Val Ser Trp Thr Tyr Asp Arg Thr Asp Leu Arg Ala
           115                 120                 125
   Asn Val Ala Tyr Asp Val Phe Thr Ala Arg Asp Pro Asp His Pro Asn
       130                 135                 140
   Trp Gly Gly Asp Tyr Glu Leu Met Ile Trp Leu Ala Arg Tyr Gly Gly
   145                 150                 155                 160
   Ile Tyr Pro Ile Gly Thr Phe His Ser Gln Val Asn Leu Ala Gly Arg
                   165                 170                 175
   Thr Trp Asp Leu Trp Thr Gly Tyr Asn Gly Asn Met Arg Val Tyr Ser
               180                 185                 190
   Phe Leu Pro Pro Ser Gly Asp Ile Arg Asp Phe Ser Cys Asp Ile Lys
           195                 200                 205
   Asp Phe Phe Asn Tyr Leu Glu Arg Asn His Gly Tyr Pro Ala Arg Glu
       210                 215                 220
   Gln Asn Leu Ile Val Tyr Gln Val Gly Thr Glu Cys Phe Thr Gly Gly
   225                 230                 235                 240
   Pro Ala Arg Phe Thr Cys Arg Asp Phe Arg Ala Asp Leu Trp
                   245                 250
<210> 15
   <211> 254
   <212> PRT
   <213> H. insolens
<400> 15
   Met Leu Lys Ser Ala Leu Leu Leu Gly Pro Ala Ala Val Ser Val Gln
    1               5                  10                  15
   Ser Ala Ser Ile Pro Thr Ile Pro Ala Asn Leu Glu Pro Arg Gln Ile
               20                  25                  30
   Arg Ser Leu Cys Glu Leu Tyr Gly Tyr Trp Ser Gly Asn Gly Tyr Glu
           35                  40                  45
   Leu Leu Asn Asn Leu Trp Gly Lys Asp Thr Ala Thr Ser Gly Trp Gln
       50                  55                  60
   Cys Thr Tyr Leu Asp Gly Thr Asn Asn Gly Gly Ile Gln Trp Ser Thr
   65                  70                  75                  80
   Ala Trp Glu Trp Gln Gly Ala Pro Asp Asn Val Lys Ser Tyr Pro Tyr
                   85                  90                  95
   Val Gly Lys Gln Ile Gln Arg Gly Arg Lys Ile Ser Asp Ile Asn Ser
               100                 105                 110
   Met Arg Thr Ser Val Ser Trp Thr Tyr Asp Arg Thr Asp Ile Arg Ala
           115                 120                 125
   Asn Val Ala Tyr Asp Val Phe Thr Ala Arg Asp Pro Asp His Pro Asn
       130                 135                 140
   Trp Gly Gly Asp Tyr Glu Leu Met Ile Trp Leu Ala Arg Tyr Gly Gly
   145                 150                 155                 160
   Ile Tyr Pro Ile Gly Thr Phe His Ser Gln Val Asn Leu Ala Gly Arg
                   165                 170                 175
   Thr Trp Asp Leu Trp Thr Gly Tyr Asn Gly Asn Met Arg Val Tyr Ser
               180                 185                 190
   Phe Leu Pro Pro Ser Gly Asp Ile Arg Asp Phe Ser Cys Asp Ile Lys
           195                 200                 205
   Asp Phe Phe Asn Tyr Leu Glu Arg Asn His Gly Tyr Pro Ala Arg Glu
       210                 215                 220
   Gln Asn Leu Ile Val Tyr Gln Val Gly Thr Glu Cys Phe Thr Gly Gly
   225                 230                 235                 240
   Pro Ala Arg Phe Thr Cys Arg Asp Phe Arg Ala Asp Leu Trp
                   245                 250
<210> 16
   <211> 247
   <212> PRT
   <213> Chaetomium brasiliense
<400> 16
   Met Lys Leu Thr Leu Val Leu Phe Val Ser Ser Leu Ala Ala Ala Thr
    1               5                  10                  15
   Pro Leu Gly Trp Arg Glu Arg Gln Gln Gln Val Ser Leu Cys Gly Gln
               20                  25                  30
   Ser Ser Ser Trp Ser Gly Asn Gly Tyr Gln Leu Asn Asn Asn Leu Trp
           35                  40                  45
   Gly Gln Ser Arg Ala Thr Ser Gly Ser Gln Cys Thr Tyr Leu Asp Ser
       50                  55                  60
   Ser Ser Asn Ser Gly Ile His Trp His Thr Thr Trp Thr Trp Glu Gly
   65                  70                  75                  80
   Gly Glu Gly Glu Val Lys Ser Tyr Ala Tyr Ser Gly Arg Gln Val Ser
                   85                  90                  95
   Thr Gly Leu Thr Ile Ala Ser Ile Asp Ser Met Gln Thr Ser Val Ser
               100                 105                 110
   Trp Glu Tyr Asn Thr Thr Asp Ile Gln Ala Asn Val Ala Tyr Asp Ile
           115                 120                 125
   Phe Thr Ala Glu Asp Pro Asp His Glu His Ser Ser Gly Asp Tyr Glu
       130                 135                 140
   Leu Met Ile Trp Leu Ala Arg Tyr Asn Asn Val Ser Pro Ile Gly Ser
   145                 150                 155                 160
   Ser Val Ala Thr Ala Thr Val Gly Gly Asp Thr Trp Asp Leu Phe Ala
                   165                 170                 175
   Gly Ala Asn Gly Asp Met Glu Val Tyr Ser Phe Val Ala Glu Asn Thr
               180                 185                 190
   Met Asn Ser Phe Ser Gly Asp Val Lys Asp Phe Phe Asp Tyr Leu Glu
           195                 200                 205
   Gln Asn Val Gly Phe Pro Val Asp Asp Gln Tyr Leu Leu Val Phe Glu
       210                 215                 220
   Leu Gly Ser Glu Ala Phe Thr Gly Gly Pro Ala Thr Leu Ser Val Ser
   225                 230                 235                 240
   Gln Phe Ser Ala Asn Ile Ala
                   245
<210> 17
   <211> 238
   <212> PRT
   <213> F. equseti
<400> 17
   Met Lys Ser Thr Leu Leu Leu Ala Gly Ala Phe Ala Pro Leu Ala Phe
    1               5                  10                  15
   Ala Lys Asp Leu Cys Glu Gln Tyr Gly Tyr Leu Ser Ser Asp Gly Tyr
               20                  25                  30
   Ser Leu Asn Asn Asn Val Trp Gly Lys Asp Ser Gly Thr Gly Asp Gln
           35                  40                  45
   Cys Thr His Val Asn Trp Asn Asn Ala Asn Gly Ala Gly Trp Asp Val
       50                  55                  60
   Glu Trp Asn Trp Ser Gly Gly Lys Asp Asn Val Lys Ser Tyr Pro Asn
   65                  70                  75                  80
   Ser Ala Leu Leu Ile Gly Glu Asp Lys Lys Thr Ile Ser Ser Ile Thr
                   85                  90                  95
   Asn Met Gln Ser Thr Ala Glu Trp Lys Tyr Ser Gly Asp Asn Leu Arg
               100                 105                 110
   Ala Asp Val Ala Tyr Asp Leu Phe Thr Ala Ala Asp Pro Asn His Glu
           115                 120                 125
   Thr Ser Ser Gly Glu Tyr Glu Leu Met Val Trp Leu Ala Arg Ile Gly
       130                 135                 140
   Gly Val Gln Pro Ile Gly Ser Leu Gln Thr Ser Val Thr Ile Glu Gly
   145                 150                 155                 160
   His Thr Trp Glu Leu Trp Val Gly Met Asn Gly Ser Met Lys Val Phe
                   165                 170                 175
   Ser Phe Val Ala Pro Thr Pro Val Asn Asn Phe Asn Ala Asp Ile Lys
               180                 185                 190
   Gln Phe Trp Asp Tyr Leu Thr Lys Ser Gln Asn Phe Pro Ala Asp Asn
           195                 200                 205
   Gln Tyr Leu Leu Thr Phe Gln Phe Gly Thr Glu Pro Phe Thr Gly Asp
       210                 215                 220
   Asn Ala Lys Phe Thr Val Thr Asn Phe Asn Ala His Leu Lys
   225                 230                 235
<210> 18
   <211> 244
   <212> PRT
   <213> F. javanicum 1
<400> 18
   Met Lys Ser Ala Ile Val Ala Ala Leu Ala Gly Leu Ala Ala Ala Ser
    1               5                  10                  15
   Pro Thr Arg Leu Ile Pro Arg Gly Gln Phe Cys Gly Gln Trp Asp Ser
               20                  25                  30
   Glu Thr Ala Gly Ala Tyr Thr Ile Tyr Asn Asn Leu Trp Gly Lys Asp
           35                  40                  45
   Asn Ala Glu Ser Gly Glu Gln Cys Thr Thr Asn Ser Gly Glu Gln Ser
       50                  55                  60
   Asp Gly Ser Ile Ala Trp Ser Val Glu Trp Ser Trp Thr Gly Gly Gln
   65                  70                  75                  80
   Gly Gln Val Lys Ser Tyr Pro Asn Ala Val Val Glu Ile Glu Lys Lys
                   85                  90                  95
   Thr Leu Gly Glu Val Ser Ser Ile Pro Ser Ala Trp Asp Trp Thr Tyr
               100                 105                 110
   Thr Gly Asn Gly Ile Ile Ala Asn Val Ala Tyr Asp Leu Phe Thr Ser
           115                 120                 125
   Ser Thr Glu Ser Gly Asp Ala Glu Tyr Glu Phe Met Ile Trp Leu Ser
       130                 135                 140
   Ala Leu Gly Gly Ala Gly Pro Ile Ser Asn Asp Gly Ser Pro Val Ala
   145                 150                 155                 160
   Thr Ala Glu Leu Ala Gly Thr Ser Trp Lys Leu Tyr Gln Gly Lys Asn
                   165                 170                 175
   Asn Gln Met Thr Val Phe Ser Phe Val Ala Glu Ser Asp Val Asn Asn
               180                 185                 190
   Phe Cys Gly Asp Leu Ala Asp Phe Thr Asp Tyr Leu Val Asp Asn His
           195                 200                 205
   Gly Val Ser Ser Ser Gln Ile Leu Gln Ser Val Gly Ala Gly Thr Glu
       210                 215                 220
   Pro Phe Glu Gly Thr Asn Ala Val Phe Thr Thr Asn Asn Tyr His Ala
   225                 230                 235                 240
   Asp Val Glu Tyr
<210> 19
   <211> 250
   <212> PRT
   <213> F. javanicum 2
<400> 19
   Met Lys Phe Phe Gly Val Val Ser Ala Ser Leu Ala Ala Thr Ala Val
    1               5                  10                  15
   Ala Thr Pro Thr Thr Pro Thr Glu Thr Ile Glu Lys Arg Asp Thr Thr
               20                  25                  30
   Trp Cys Asp Ala Phe Gly Ser Leu Ala Thr Ser Gly Tyr Thr Val Tyr
           35                  40                  45
   His Asn Asn Trp Gly Lys Gly Asp Ala Thr Ser Gly Ser Gln Cys Thr
       50                  55                  60
   Thr Phe Thr Ser Val Ser Asn Asn Asn Phe Val Trp Ser Thr Ser Trp
   65                  70                  75                  80
   Thr Trp Ala Gly Gly Ala Gly Lys Val Lys Ser Tyr Ser Asn Val Ala
                   85                  90                  95
   Leu Glu Lys Ile Asn Lys Lys Ile Ser Asp Ile Lys Ser Val Ser Thr
               100                 105                 110
   Arg Trp Ile Trp Arg Tyr Thr Gly Thr Lys Met Ile Ala Asn Val Ser
           115                 120                 125
   Tyr Asp Leu Trp Phe Ala Pro Thr Ala Ser Ser Asn Asn Ala Tyr Glu
       130                 135                 140
   Ile Met Ile Trp Val Gly Ala Tyr Gly Gly Ala Leu Pro Ile Ser Thr
   145                 150                 155                 160
   Pro Gly Lys Gly Val Ile Asp Arg Pro Thr Leu Ala Gly Ile Pro Trp
                   165                 170                 175
   Asp Val Tyr Lys Gly Pro Asn Gly Asp Val Thr Val Ile Ser Phe Val
               180                 185                 190
   Ala Ser Ser Asn Gln Gly Asn Phe Gln Ala Asp Leu Lys Glu Phe Leu
           195                 200                 205
   Asn Tyr Leu Thr Ser Lys Gln Gly Leu Pro Ser Asn Tyr Val Ala Thr
       210                 215                 220
   Ser Phe Gln Ala Gly Thr Glu Pro Phe Glu Gly Thr Asn Ala Val Leu
    225                 230                 235                 240
   Lys Thr Ser Ala Tyr Thr Ile Ser Val Asn
                   245                 250
<210> 20
   <211> 238
   <212> PRT
   <213> G. roseum 1
<400> 20
   Met Lys Ala Asn Ile Val Ile Leu Ser Leu Phe Ala Pro Leu Ala Ala
   1 5 10 15
   Val Ala Gln Thr Leu Cys Gly Gln Tyr Ser Ser Asn Thr Gln Gly Gly
   20 25 30
   Tyr Ile Phe Asn Asn Asn Met Trp Gly Met Gly Ser Gly Ser Gly Ser
   35 40 45
   Gln Cys Thr Tyr Val Asp Lys Val Trp Ala Glu Gly Val Ala Trp His
   50 55 60
   Thr Asp Trp Ser Trp Ser Gly Gly Asp Asn Asn Val Lys Ser Tyr Pro
   65 70 75 80
   Tyr Ser Gly Arg Glu Leu Gly Thr Lys Arg Ile Val Ser Ser Ile Lys
   85 90 95
   Ser Ile Ser Ser Gly Ala Asp Trp Asp Tyr Thr Gly Ser Asn Leu Arg
   100 105 110
   Ala Asn Ala Ala Tyr Asp Ile Phe Thr Ser Ala Asn Pro Asn His Ala
   115 120 125
   Thr Ser Ser Gly Asp Tyr Glu Val Met Ile Trp Leu Ala Asn Leu Gly
   130 135 140
   Gly Leu Thr Pro Ile Gly Ser Pro Ile Gly Thr Val Lys Ala Ala Gly
   145 150 155 160
   Arg Asp Trp Glu Leu Trp Asp Gly Tyr Asn Gly Ala Met Arg Val Tyr
   165 170 175
   Ser Phe Val Ala Pro Ser Gln Leu Asn Ser Phe Asp Gly Glu Ile Met
   180 185 190
   Asp Phe Phe Tyr Val Val Lys Asp Met Arg Gly Phe Pro Ala Asp Ser
   195 200 205
   Gln His Leu Leu Thr Val Gln Phe Gly Thr Glu Pro Ile Ser Gly Ser
   210 215 220
   Gly Ala Lys Phe Ser Val Ser His Trp Ser Ala Lys Leu Gly
   225 230 235
<210> 21
   <211> 348
   <212> PRT
   <213> G. roseum 2
<400> 21
   Met Lys Ser Ile Ile Ser Phe Phe Gly Leu Ala Thr Leu Val Ala Ala
   1 5 10 15
   Ala Pro Ser Gln Asn Pro Thr Arg Thr Gln Pro Leu Glu Lys Arg Ala
   20 25 30
   Thr Thr Leu Cys Gly Gln Trp Asp Ser Val Glu Thr Gly Gly Tyr Thr
   35 40 45
   Ile Tyr Asn Asn Leu Trp Gly Gln Asp Asn Gly Ser Gly Ser Gln Cys
   50 55 60
   Leu Thr Val Glu Gly Val Thr Asp Gly Leu Ala Ala Trp Ser Ser Thr
   65 70 75 80
   Trp Ser Trp Ser Gly Gly Ser Ser Ser Val Lys Ser Tyr Ser Asn Ala
   85 90 95
   Val Leu Ser Ala Glu Ala Ala Arg Ile Ser Ala Ile Ser Ser Ile Pro
               100                 105                 110
   Ser Lys Trp Glu Trp Ser Tyr Thr Gly Thr Asp Ile Val Ala Asn Val
           115                 120                 125
   Ala Tyr Asp Leu Phe Ser Asn Thr Asp Cys Gly Asp Thr Pro Glu Tyr
       130                 135                 140
   Glu Ile Met Ile Trp Leu Ser Ala Leu Gly Gly Ala Gly Pro Ile Ser
   145                 150                 155                 160
   Ser Thr Gly Ser Ser Ile Ala Thr Val Thr Ile Ala Gly Ala Ser Trp
                   165                 170                 175
   Asn Leu Trp Gln Gly Gln Asn Asn Gln Met Ala Val Phe Ser Phe Val
               180                 185                 190
   Ala Glu Ser Asp Gln Lys Ser Phe Ser Gly Asp Leu Asn Asp Phe Ile
           195                 200                 205
   Gln Tyr Leu Val Asp Ser Gln Gly Tyr Ser Gly Ser Gln Cys Leu Tyr
       210                 215                 220
   Ser Ile Gly Ala Gly Thr Glu Pro Phe Thr Gly Thr Asp Ala Glu Phe
   225                 230                 235                 240
   Ile Thr Thr Gly Tyr Ser Val Ser Val Ser Ala Gly Asp Ser Gly Cys
                   245                 250                 255
   Asp Glu Thr Thr Thr Ser Ser Gln Ala Gln Ser Ser Thr Val Glu Thr
               260                 265                 270
   Ser Thr Ala Thr Gln Pro Gln Ser Ser Ser Thr Val Val Pro Thr Val
           275                 280                 285
   Thr Leu Ser Gln Pro Ser Asn Glu Ser Thr Thr Thr Pro Val Gln Ser
       290                 295                 300
   Gln Pro Ser Ser Val Glu Thr Thr Pro Thr Ala Gln Pro Gln Ser Ser
   305                 310                 315                 320
   Ser Val Gln Thr Thr Thr Thr Ala Gln Ala Gln Pro Thr Ser Gly Thr
                   325                 330                 335
   Gly Cys Ser Arg Arg Arg Lys Arg Arg Ala Val Val
               340                 345
<210> 22
   <211> 236
   <212> PRT
   <213> G. roseum 3
<400> 22
   Met Lys Phe Gln Leu Leu Ser Leu Thr Ala Phe Ala Pro Leu Ser Leu
    1               5                  10                  15
   Ala Ala Leu Cys Gly Gln Tyr Gln Ser Gln Ser Gln Gly Gly Tyr Ile
               20                  25                  30
   Phe Asn Asn Asn Lys Trp Gly Gln Gly Ser Gly Ser Gly Ser Gln Cys
           35                  40                  45
   Leu Thr Ile Asp Lys Thr Trp Asp Ser Asn Val Ala Phe His Ala Asp
       50                  55                  60
   Trp Ser Trp Ser Gly Gly Thr Asn Asn Val Lys Ser Tyr Pro Asn Ala
   65                  70                  75                  80
   Gly Leu Glu Phe Ser Arg Gly Lys Lys Val Ser Ser Ile Gly Thr Ile
                   85                  90                  95
   Asn Gly Gly Ala Asp Trp Asp Tyr Ser Gly Ser Asn Ile Arg Ala Asn
               100                 105                 110
   Val Ala Tyr Gly Ile Phe Thr Ser Ala Asp Pro Asn His Val Thr Ser
           115                 120                 125
   Ser Gly Asp Tyr Glu Leu Met Ile Trp Leu Gly Lys Leu Gly Asp Ile
       130                 135                 140
   Tyr Pro Ile Gly Asn Ser Ile Gly Arg Val Glu Ala Ala Asn Arg Glu
   145                 150                 155                 160
   Trp Asp Phe Leu Val Gly Tyr Asn Gly Ala Met Lys Val Phe Ser Phe
                   165                 170                 175
   Val Ala Pro Ser Pro Val Thr Leu Phe Asp Gly Asn Ile Met Asp Phe
               180                 185                 190
   Phe Tyr Val Met Arg Asp Met Gln Gly Tyr Pro Met Asp Lys Gln Tyr
           195                 200                 205
   Leu Leu Ser Leu Gln Phe Gly Thr Glu Pro Phe Thr Gly Ser Asn Ala
       210                 215                 220
   Asn Phe Ser Cys Trp Tyr Phe Gly Ala Lys Ile Lys
   225                 230                 235
<210> 23
   <211> 237
   <212> PRT
   <213> G. roseum 4
<400> 23
   Met Lys Thr Gly Ile Ala Tyr Leu Ala Ala Val Leu Pro Leu Ala Met
    1               5                  10                  15
   Ala Glu Ser Leu Cys Asp Gln Tyr Ala Tyr Leu Ser Arg Asp Gly Tyr
               20                  25                  30
   Asn Phe Asn Asn Asn Glu Trp Gly Ala Ala Thr Gly Thr Gly Asp Gln
           35                  40                  45
   Cys Thr Tyr Val Asp Ser Thr Ser Ser Gly Gly Val Ser Trp His Ser
       50                  55                  60
   Asp Trp Thr Trp Ser Gly Ser Glu Ser Glu Ile Lys Ser Tyr Pro Tyr
   65                  70                  75                  80
   Ser Gly Leu Asp Leu Pro Glu Lys Lys Ile Val Thr Ser Ile Gly Ser
                   85                  90                  95
   Ile Ser Thr Gly Ala Glu Trp Ser Tyr Ser Gly Ser Asp Ile Arg Ala
               100                 105                 110
   Asp Val Ala Tyr Asp Thr Phe Thr Ala Ala Asp Pro Asn His Ala Thr
           115                 120                 125
   Ser Ser Gly Asp Tyr Glu Val Met Ile Trp Leu Ala Asn Leu Gly Gly
       130                 135                 140
   Leu Thr Pro Ile Gly Ser Pro Ile Gly Thr Val Lys Ala Ala Gly Arg
   145                 150                 155                 160
   Asp Trp Glu Leu Trp Asp Gly Tyr Asn Gly Ala Met Arg Val Tyr Ser
                   165                 170                 175
   Phe Val Ala Pro Ser Gln Leu Asn Ser Phe Asp Gly Glu Ile Met Asp
               180                 185                 190
   Phe Phe Tyr Val Val Lys Asp Met Arg Gly Phe Pro Ala Asp Ser Gln
           195                 200                 205
   His Leu Leu Thr Val Gln Phe Gly Thr Glu Pro Ile Ser Gly Ser Gly
       210                 215                 220
   Ala Lys Phe Ser Val Ser His Trp Ser Ala Lys Leu Gly
   225                 230                 235
<210> 24
   <211> 237
   <212> PRT
   <213> Memnoniella echinata
<400> 24
   Met Lys Val Ala Ala Leu Leu Val Ala Leu Ser Pro Leu Ala Phe Ala
    1               5                  10                  15
   Gln Ser Leu Cys Asp Gln Tyr Ser Tyr Tyr Ser Ser Asn Gly Tyr Glu
               20                  25                  30
   Phe Asn Asn Asn Met Trp Gly Arg Asn Ser Gly Gln Gly Asn Gln Cys
           35                      40                      45
   Thr Tyr Val Asp Tyr Ser Ser Pro Asn Gly Val Gly Trp Arg Val Asn
       50                      55                      60
   Trp Asn Trp Ser Gly Gly Asp Asn Asn Val Lys Ser Tyr Pro Tyr Ser
   65                      70                      75                  80
   Gly Arg Gln Leu Pro Thr Lys Arg Ile Val Ser Trp Ile Gly Ser Leu
                   85                      90                      95
   Pro Thr Thr Val Ser Trp Asn Tyr Gln Gly Asn Asn Leu Arg Ala Asn
               100                     105                     110
   Val Ala Tyr Asp Leu Phe Thr Ala Ala Asn Pro Asn His Pro Asn Ser
           115                     120                     125
   Ser Gly Asp Tyr Glu Leu Met Ile Trp Leu Gly Arg Leu Gly Asn Val
       130                     135                     140
   Tyr Pro Ile Gly Asn Gln Val Ala Thr Val Asn Ile Ala Gly Gln Gln
   145                     150                     155                 160
   Trp Asn Leu Tyr Tyr Gly Tyr Asn Gly Ala Met Gln Val Tyr Ser Phe
                   165                     170                     175
   Val Ser Pro Asn Gln Leu Asn Tyr Phe Ser Gly Asn Val Lys Asp Phe
               180                     185                     190
   Phe Thr Tyr Leu Gln Tyr Asn Arg Ala Tyr Pro Ala Asp Ser Gln Tyr
           195                     200                     205
   Leu Ile Thr Tyr Gln Phe Gly Thr Glu Pro Phe Thr Gly Gln Asn Ala
       210                     215                     220
   Val Phe Thr Val Ser Asn Trp Ser Ala Gln Gln Asn Asn
   225                     230                     235
<210> 25
   <211> 245
   <212> PRT
   <213> Emericella desertoru
<400> 25
   Met Lys Leu Leu Ala Leu Ser Leu Val Ser Leu Ala Ser Ala Ala Ser
    1               5                  10                  15
   Ala Ala Ser Ile Leu Ser Asn Thr Phe Thr Arg Arg Ser Asp Phe Cys
               20                  25                  30
   Gly Gln Trp Asp Thr Ala Thr Val Gly Asn Phe Ile Val Tyr Asn Asn
           35                  40                  45
   Leu Trp Gly Gln Asp Asn Ala Asp Ser Gly Ser Gln Thr Gly Val Asp
       50                  55                  60
   Ser Ala Asn Gly Asn Ser Ile Ser Trp His Thr Thr Trp Ser Trp Ser
   65                  70                  75                  80
   Gly Gly Ser Ser Ser Val Lys Ser Tyr Ala Asn Ala Ala Tyr Gln Phe
                   85                  90                  95
   Thr Ser Thr Lys Leu Asn Ser Leu Ser Ser Ile Pro Thr Ser Trp Lys
               100                 105                 110
   Trp Gln Tyr Ser Thr Thr Asp Ile Val Ala Asn Val Ala Tyr Asp Leu
           115                 120                 125
   Phe Thr Ser Ser Ser Ala Gly Gly Asp Ser Glu Tyr Glu Ile Met Ile
       130                 135                 140
   Trp Leu Ala Ala Leu Gly Gly Ala Gly Pro Ile Ser Ser Thr Gly Ser
   145                 150                 155                 160
   Ser Ile Ala Thr Val Thr Leu Gly Gly Val Thr Trp Ser Leu Tyr Ser
                   165                 170                 175
   Gly Pro Asn Gly Ser Met Gln Val Tyr Ser Phe Val Ala Ser Ser Thr
               180                 185                 190
   Thr Glu Ser Phe Ser Ala Asp Leu Met Asp Phe Ile Asn Tyr Leu Ala
           195                 200                 205
   Glu Asn Gln Gly Leu Ser Ser Ser Gln Leu Thr His Val Gln Ala Gly
       210                 215                 220
   Thr Glu Pro Phe Thr Gly Thr Asp Ala Thr Leu Thr Val Ser Ser Tyr
   225                 230                 235                 240
   Ser Val Ser Val Ser
                   245
<210> 26
   <211> 371
   <212> PRT
   <213> Actinomycete 11AG8
<400> 26
   Met Arg Ser His Pro Arg Ser Ala Thr Met Thr Val Leu Val Val Leu
    1               5                  10                  15
   Ala Ser Leu Gly Ala Leu Leu Thr Ala Ala Ala Pro Ala Gln Ala Asn
               20                  25                  30
   Gln Gln Ile Cys Asp Arg Tyr Gly Thr Thr Thr Ile Gln Asp Arg Tyr
           35                  40                  45
   Val Val Gln Asn Asn Arg Trp Gly Thr Ser Ala Thr Gln Cys Ile Asn
       50                  55                  60
   Val Thr Gly Asn Gly Phe Glu Ile Thr Gln Ala Asp Gly Ser Val Pro
   65                  70                  75                  80
   Thr Asn Gly Ala Pro Lys Ser Tyr Pro Ser Val Tyr Asp Gly Cys His
                   85                  90                  95
   Tyr Gly Asn Cys Ala Pro Arg Thr Thr Leu Pro Met Arg Ile Ser Ser
               100                 105                 110
   Ile Gly Ser Ala Pro Ser Ser Val Ser Tyr Arg Tyr Thr Gly Asn Gly
           115                 120                 125
   Val Tyr Asn Ala Ala Tyr Asp Ile Trp Leu Asp Pro Thr Pro Arg Thr
       130                 135                 140
   Asn Gly Val Asn Arg Thr Glu Ile Met Ile Trp Phe Asn Arg Val Gly
   145                 150                 155                 160
   Pro Val Gln Pro Ile Gly Ser Pro Val Gly Thr Ala His Val Gly Gly
                   165                 170                 175
   Arg Ser Trp Glu Val Trp Thr Gly Ser Asn Gly Ser Asn Asp Val Ile
               180                 185                 190
   Ser Phe Leu Ala Pro Ser Ala Ile Ser Ser Trp Ser Phe Asp Val Lys
           195                 200                 205
   Asp Phe Val Asp Gln Ala Val Ser His Gly Leu Ala Thr Pro Asp Trp
       210                 215                 220
   Tyr Leu Thr Ser Ile Gln Ala Gly Phe Glu Pro Trp Glu Gly Gly Thr
   225                 230                 235                 240
   Gly Leu Ala Val Asn Ser Phe Ser Ser Ala Val Asn Ala Gly Gly Gly
                   245                 250                 255
   Asn Gly Gly Thr Pro Gly Thr Pro Ala Ala Cys Gln Val Ser Tyr Ser
               260                 265                 270
   Thr His Thr Trp Pro Gly Gly Phe Thr Val Asp Thr Thr Ile Thr Asn
           275                 280                 285
   Thr Gly Ser Thr Pro Val Asp Gly Trp Glu Leu Asp Phe Thr Leu Pro
       290                 295                 300
   Ala Gly His Thr Val Thr Ser Ala Trp Asn Ala Leu Ile Ser Pro Ala
   305                 310                 315                 320
   Ser Gly Ala Val Thr Ala Arg Ser Thr Gly Ser Asn Gly Arg Ile Ala
                   325                 330                 335
   Ala Asn Gly Gly Thr Gln Ser Phe Gly Phe Gln Gly Thr Ser Ser Gly
               340                 345                 350
   Thr Gly Phe Asn Ala Pro Ala Gly Gly Arg Leu Asn Gly Thr Ser Cys
           355                 360                 365
   Thr Val Arg
       370
<210> 27
   <211> 381
   <212> PRT
   <213> S. lividans CelB
<400> 27
   Met Arg Thr Leu Arg Pro Gln Ala Arg Ala Pro Arg Gly Leu Leu Ala
    1               5                  10                  15
   Ala Leu Gly Ala Val Leu Ala Ala Phe Ala Leu Val Ser Ser Leu Val
               20                  25                  30
   Thr Ala Ala Ala Pro Ala Gln Ala Asp Thr Thr Ile Cys Glu Pro Phe
           35                  40                  45
   Gly Thr Thr Thr Ile Gln Gly Arg Tyr Val Val Gln Asn Asn Arg Trp
       50                  55                  60
   Gly Ser Thr Ala Pro Gln Cys Val Thr Ala Thr Asp Thr Gly Phe Arg
   65                  70                  75                  80
   Val Thr Gln Ala Asp Gly Ser Ala Pro Thr Asn Gly Ala Pro Lys Ser
                   85                  90                  95
   Tyr Pro Ser Val Phe Asn Gly Cys His Tyr Thr Asn Cys Ser Pro Gly
               100                 105                 110
   Thr Asp Leu Pro Val Arg Leu Asp Thr Val Ser Ala Ala Pro Ser Ser
           115                 120                 125
   Ile Ser Tyr Gly Phe Val Asp Gly Ala Val Tyr Asn Ala Ser Tyr Asp
       130                 135                 140
   Ile Trp Leu Asp Pro Thr Ala Arg Thr Asp Gly Val Asn Gln Thr Glu
   145                 150                 155                 160
   Ile Met Ile Trp Phe Asn Arg Val Gly Pro Ile Gln Pro Ile Gly Ser
                   165                 170                 175
   Pro Val Gly Thr Ala Ser Val Gly Gly Arg Thr Trp Glu Val Trp Ser
               180                 185                 190
   Gly Gly Asn Gly Ser Asn Asp Val Leu Ser Phe Val Ala Pro Ser Ala
           195                 200                 205
   Ile Ser Gly Trp Ser Phe Asp Val Met Asp Phe Val Arg Ala Thr Val
       210                 215                 220
   Ala Arg Gly Leu Ala Glu Asn Asp Trp Tyr Leu Thr Ser Val Gln Ala
   225                 230                 235                 240
   Gly Phe Glu Pro Trp Gln Asn Gly Ala Gly Leu Ala Val Asn Ser Phe
                   245                 250                 255
   Ser Ser Thr Val Glu Thr Gly Thr Pro Gly Gly Thr Asp Pro Gly Asp
               260                 265                 270
   Pro Gly Gly Pro Ser Ala Cys Ala Val Ser Tyr Gly Thr Asn Val Trp
           275                 280                 285
   Gln Asp Gly Phe Thr Ala Asp Val Thr Val Thr Asn Thr Gly Thr Ala
       290                 295                 300
   Pro Val Asp Gly Trp Gln Leu Ala Phe Thr Leu Pro Ser Gly Gln Arg
   305                 310                 315                 320
   Ile Thr Asn Ala Trp Asn Ala Ser Leu Thr Pro Ser Ser Gly Ser Val
                   325                 330                 335
   Thr Ala Thr Gly Ala Ser His Asn Ala Arg Ile Ala Pro Gly Gly Ser
               340                 345                 350
   Leu Ser Phe Gly Phe Gln Gly Thr Tyr Gly Gly Ala Phe Ala Glu Pro
           355                 360                 365
   Thr Gly Phe Arg Leu Asn Gly Thr Ala Cys Thr Thr Val
       370                 375                 380
<210> 28
   <211> 260
   <212> PRT
   <213> Rhodothermus marinus
<400> 28
   Met Asn Val Met Arg Ala Val Leu Val Leu Ser Leu Leu Leu Leu Phe
   1               5                   10                  15
   Gly Cys Asp Trp Leu Phe Pro Asp Gly Asp Asn Gly Lys Glu Pro Glu
               20                  25                  30
   Pro Glu Pro Glu Pro Thr Val Glu Leu Cys Gly Arg Trp Asp Ala Arg
           35                  40                  45
   Asp Val Ala Gly Gly Arg Tyr Arg Val Ile Asn Asn Val Trp Gly Ala
       50                  55                  60
   Glu Thr Ala Gln Cys Ile Glu Val Gly Leu Glu Thr Gly Asn Phe Thr
   65                  70                  75                  80
   Ile Thr Arg Ala Asp His Asp Asn Gly Asn Asn Val Ala Ala Tyr Pro
                   85                  90                  95
   Ala Ile Tyr Phe Gly Cys His Trp Ala Pro Ala Arg Ala Ile Arg Asp
               100                 105                 110
   Cys Ala Ala Arg Ala Gly Ala Val Arg Arg Ala His Glu Leu Asp Val
           115                 120                 125
   Thr Pro Ile Thr Thr Gly Arg Trp Asn Ala Ala Tyr Asp Ile Trp Phe
       130                 135                 140
   Ser Pro Val Thr Asn Ser Gly Asn Gly Tyr Ser Gly Gly Ala Glu Leu
   145                 150                 155                 160
   Met Ile Trp Leu Asn Trp Asn Gly Gly Val Met Pro Gly Gly Ser Arg
                   165                 170                 175
   Val Ala Thr Val Glu Leu Ala Gly Ala Thr Trp Glu Val Trp Tyr Ala
               180                 185                 190
   Asp Trp Asp Trp Asn Tyr Ile Ala Tyr Arg Arg Thr Thr Pro Thr Thr
           195                 200                 205
   Ser Val Ser Glu Leu Asp Leu Lys Ala Phe Ile Asp Asp Ala Val Ala
       210                 215                 220
   Arg Gly Tyr Ile Arg Pro Glu Trp Tyr Leu His Ala Val Glu Thr Gly
   225                 230                 235                 240
   Phe Glu Leu Trp Glu Gly Gly Ala Gly Leu Arg Thr Ala Asp Phe Ser
                   245                 250                 255
   Val Thr Val Gln
               260
<210> 29
   <211> 264
   <212> PRT
   <213> Erwinia carot
<400> 29
   Met Gln Thr Val Asn Thr Gln Pro His Arg Ile Phe Arg Val Leu Leu
   1 5 10 15
   Pro Ala Val Phe Ser Ser Leu Leu Leu Ser Ser Leu Thr Val Ser Ala
   20 25 30
   Ala Ser Ser Ser Asn Asp Ala Asp Lys Leu Tyr Phe Gly Asn Asn Lys
   35 40 45
   Tyr Tyr Leu Phe Asn Asn Val Trp Gly Lys Asp Glu Ile Lys Gly Trp
   50 55 60
   Gln Gln Thr Ile Phe Tyr Asn Ser Pro Ile Ser Met Gly Trp Asn Trp
   65 70 75 80
   His Trp Pro Ser Ser Thr His Ser Val Lys Ala Tyr Pro Ser Leu Val
   85 90 95
   Ser Gly Trp His Trp Thr Ala Gly Tyr Thr Glu Asn Ser Gly Leu Pro
   100 105 110
   Ile Gln Leu Ser Ser Asn Lys Ser Ile Thr Ser Asn Val Thr Tyr Ser
   115 120 125
   Ile Lys Ala Thr Gly Thr Tyr Asn Ala Ala Tyr Asp Ile Trp Phe His
   130 135 140
   Thr Thr Asp Lys Ala Asn Trp Asp Ser Ser Pro Thr Asp Glu Leu Met
   145 150 155 160
   Ile Trp Leu Asn Asp Thr Asn Ala Gly Pro Ala Gly Asp Tyr Ile Glu
   165 170 175
   Thr Val Phe Leu Gly Asp Ser Ser Trp Asn Val Phe Lys Gly Trp Ile
   180 185 190
   Asn Ala Asp Asn Gly Gly Gly Trp Asn Val Phe Ser Phe Val His Thr
   195 200 205
   Ser Gly Thr Asn Ser Ala Ser Leu Asn Ile Arg His Phe Thr Asp Tyr
   210 215 220
   Leu Val Gln Thr Lys Gln Trp Met Ser Asp Glu Lys Tyr Ile Ser Ser
   225 230 235 240
   Val Glu Phe Gly Thr Glu Ile Phe Gly Gly Asp Gly Gln Ile Asp Ile
   245 250 255
   Thr Glu Trp Arg Val Asp Val Lys
   260
<210> 30
   <211> 14
   <212> PRT
   <213> Artificial Sequence
<220>
   <223> Synthetic
<400> 30
   Ala Ala Tyr Ala Ala Tyr Tyr Thr Asn Thr Gly Gly Gly Gly
   1 5 10
<210> 31
   <211> 14
   <212> PRT
   <213> Artificial Sequence
<220>
   <223> Synthetic
<400> 31
   Cys Ala Arg Ala Ala Tyr Tyr Thr Asn Thr Gly Gly Gly Gly
   1 5 10
<210> 32
   <211> 17
   <212> PRT
   <213> Artificial Sequence
<220>
   <223> Synthetic
<400> 32
   Ala Ala Tyr Ala Ala Tyr Ala Ala Tyr His Trp Ile Thr Gly Gly Gly
   1 5 10 15
   Gly
<210> 33
   <211> 15
   <212> PRT
   <213> Artificial Sequence
<220>
   <223> Synthetic
<400> 33
   Gly Ala Arg Tyr Thr Asn Ala Thr Gly Ala Thr His Thr Gly Gly
   1 5 10 15
<210> 34
   <211> 15
   <212> PRT
   <213> Artificial Sequence
<220>
   <223> Synthetic
<400> 34
   Cys Cys Ala Asp Ala Thr Cys Ala Thr Asn Ala Arg Tyr Thr Cys
   1 5 10 15
<210> 35
   <211> 18
   <212> PRT
   <213> Artificial Sequence
<220>
   <223> Synthetic
<400> 35
   Thr Ala Tyr Gly Ala Arg Tyr Thr Ile Ala Thr Gly Ala Thr His Thr
   1               5                   10                  15
   Gly Gly
<210> 36
   <211> 18
   <212> PRT
   <213> Artificial Sequence
<220>
   <223> Synthetic
<400> 36
   Cys Cys Ala Asp Ala Thr Cys Ala Thr Ile Ala Arg Tyr Thr Cys Arg
   1               5                   10                  15
   Thr Ala
<210> 37
   <211> 17
   <212> PRT
   <213> Artificial Sequence
<220>
   <223> Synthetic
<400> 37
   Gly Thr Arg Ala Ala Asn Gly Gly Tyr Thr Cys Arg Gly Thr Arg Cys
   1               5                   10                  15
   Cys
<210> 38
   <211> 17
   <212> PRT
   <213> Artificial Sequence
<220>
   <223> Synthetic
<400> 38
   Gly Thr Arg Ala Ala Asn Gly Gly Tyr Thr Cys Arg Gly Thr Tyr Cys
   1               5                   10                  15
   Cys
<210> 39
   <211> 17
   <212> PRT
   <213> Artificial Sequence
<220>
   <223> Synthetic
<400> 39
   Gly Thr Arg Ala Ala Asn Gly Gly Tyr Thr Cys Tyr Gly Thr Arg Cys
    1               5                  10                  15
   Cys
<210> 40
   <211> 17
   <212> PRT
   <213> Artificial Sequence
<220>
   <223> Synthetic
<400> 40
   Gly Thr Arg Ala Ala Asn Gly Gly Tyr Thr Cys Tyr Gly Thr Tyr Cys
    1               5                  10                  15
   Cys
<210> 41
   <211> 17
   <212> PRT
   <213> Artificial Sequence
<220>
   <223> Synthetic
<400> 41
   Gly Thr Arg Ala Ala Arg Cys Ala Tyr Thr Cys Asn Gly Thr Asn Cys
    1               5                  10                  15
   Cys